# EUROPEAN PATENT APPLICATION

(11) **EP 4 570 304 A1**
(43) Date of publication of application: **18.06.2025**
(21) Application number: 23851637.1
(22) Date of filing: 31.07.2023
(51) Int. Cl.: A61N 1/365, A61N 1/362, A61N 1/372

(54) **MEDICAL INSTRUMENT FOR DELIVERING PULSE STIMULATION**

(30) Priority: 08.08.2022 CN 202210944885; 08.08.2022 CN 202210944820
(71) Applicant: United Innomed (Shanghai) Limited, Shanghai 201315 (CN)
(72) Inventor: WANG, Li, Shanghai 201315 (CN)
(74) Representative: dompatent von Kreisler Selting Werner - Partnerschaft von Patent- und Rechtsanwälten mbB
(86) International application number: PCT/CN2023/110350
(87) International publication number: WO 2024/032416

(57) **Abstract**

Disclosed is a medical instrument for delivering pulse stimulation. The medical instrument comprises at least one ventricular electrode wire and a control device. The ventricular electrode wire is configured to be arranged at a myocardial position, and the control device is configured to execute a pulse stimulation control method. The pulse stimulation control method comprises the following steps: acquiring a far-field electrocardiogram and an in-vivo near-field myocardial electrocardiogram corresponding to the myocardial position; and according to an R wave in the far-field electrocardiogram and an R wave in the in-vivo near-field myocardial electrocardiogram, determining whether CCM pulse stimulation is delivered to the myocardial position or not. The medical instrument for delivering pulse stimulation provided in the present invention can ensure the timeliness, safety and effectiveness of CCM pulse stimulation to the heart of a patient.

## Description

The present application claims the right of priorities of Chinese patent application CN202210944885.5 with a filing date of August 8, 2022, and Chinese patent application CN202210944820.0 with a filing date of August 8, 2022. The contents of the above Chinese patent applications are incorporated herein by reference in their entirety.

### TECHNICAL FIELD

The present disclosure relates to the technical field of medical instruments, and in particular, to a medical instrument for delivering pulse stimulation.

### BACKGROUND

The existing medical apparatus with Cardiac Contractility Modulation (CCM) functionality in the market is primarily used for patients with chronic heart failure. It typically involves the implantation of two bipolar electrode leads into the right ventricular septum to sense local myocardial potentials and deliver pulse stimulation at a certain time after the sensing to enhance ventricular myocardial contractility.

In addition, this medical apparatus is currently only capable of providing CCM therapy. However, a significant portion of patients with CCM indications (EF < 35%) also require primary prevention of SCD (sudden cardiac death) and thus need implantation of an ICD (implantable cardioverter-defibrillator). At present, these patients with heart failure receive both a CCM device and an ICD (implantable cardioverter-defibrillator) as two implantable instruments. On the contrary, many patients who are currently receiving ICD therapy also have indications of CCM therapy, and they can benefit from CCM therapy by improving heart failure symptoms and hemodynamics through the CCM device. Currently, most ICD patients benefit from SCD prevention after ICD implantation, without receiving CCM therapy for heart failure. Additionally, the current CCM instruments do not provide pacing therapy for bradycardia, which may also be a therapy needed by some CCM patients.

One reason is that the current CCM system (instrument with at least two ventricular electrode leads) is relatively complex. This system requires two bipolar ventricular electrode leads placed at the right ventricular septum to sense local myocardial activation and a time sequence of two sensing events, ensuring that ventricular activation originates from the atrium, rather than from the ventricle itself (including ventricular pacing). One or both of the electrode leads are also used to deliver CCM pulses during the absolute refractory period of the local myocardium. CCM stimulation is not directly delivered to the left ventricle (LV) myocardium, while the LV is typically the chamber most in need of enhanced contractility. Although it has been shown that CCM has an overall effect on cardiac contractility and cardiac function, studies indicate that this effect starts at the site of stimulation and only partially affects overall cardiac contractility or the LV.

Currently, the intravenous ICD systems, i.e., traditional ICD systems, are available on the market, where a single-chamber ICD has only one lead in the right ventricle (RV), a dual-chamber ICD has only two leads (one in the right atrium (RA) and one in the RV), and three leads (one in the RA, one in the RV, and one on the epicardial surface of the left ventricle) are used for CRT-D (Cardiac Resynchronization Therapy Defibrillator) instruments. These three systems have the following in common: 1) There is only one lead in the RV, unlike the CCM instrument which has two leads; 2) The RV lead has a defibrillation coil electrode in the RV, which forms a defibrillation circuit with the ICD housing. Sometimes there is a second defibrillation coil electrode on the site corresponding to the superior vena cava (SVC), and both are located on the RV lead. One of the reasons that ICDs cannot currently provide CCM therapy on their own is that there is no certainty that CCM stimulation in the local myocardium is absolutely safe and will not trigger malignant ventricular arrhythmias. Currently, CCM instruments cannot provide pacing therapy, while some patients with heart failure can benefit from pacing and increasing their heart rate.

### CONTENT OF THE PRESENT INVENTION

One of the technical problems to be solved by the present disclosure is to provide, in order to overcome three major limitations in the prior art of only being able to deliver CCM pulse stimulation when ventricular electrical activity is conducted from the atrium, having to sense local activation of the right ventricular septum myocardium and a time sequence of two sensing events with pairs of electrodes on two ventricular electrode leads in order to judge whether a pulse stimulation can be delivered, as well as delivering pulse stimulation only in the right ventricular septum, a medical instrument capable of ensuring that CCM pulse stimulation is delivered during a ventricular deliverable period without the above-mentioned limitations.

Another technical problem to be solved by the present disclosure is to provide a medical instrument for delivering pulse stimulation, which can implement the CCM therapy function using its own ventricular electrode lead.

Another technical problem to be solved by the present disclosure is to provide a medical instrument for delivering pulse stimulation in order to overcome the problems that existing CCM instruments are not able to provide ICD therapy to patients in need of SCD prevention, which results in the need to implant both CCM and ICD medical instruments in the patient leading to an increase in the surgical and/or post-surgical risks (e.g., infections), or only one of the therapies can be chosen due to the financial burden. This solution enables patients with heart failure to receive required therapies (mitigating heart failure and preventing sudden cardiac death), reduces comfort complexity and the financial burden on patients, and significantly improves the cost-effectiveness of the implanted instrument.

Another technical problem to be solved by the present disclosure is to provide a safer and more reliable pulse stimulation control method in order to overcome the problem in the prior art of delivering CCM pulse stimulation in an erroneous situation due to false sensing of an R wave in a far-field electrocardiogram or an *in vivo* near-field electrocardiogram.

The present disclosure solves the above technical problems through the following technical solutions:
The present disclosure provides a medical instrument for delivering pulse stimulation, comprising at least one ventricular electrode lead and a control device, the ventricular electrode lead being configured to be arranged at a myocardial position of a ventricle; the control device being configured to execute a pulse stimulation control method, and the pulse stimulation control method comprising the following steps:
acquiring a far-field electrocardiogram and an *in vivo* near-field myocardial electrocardiogram corresponding to the myocardial position; and
determining whether to deliver CCM pulse stimulation to the myocardial position via the ventricular electrode lead according to an R wave in the far-field electrocardiogram and an R wave in the *in vivo* near-field myocardial electrocardiogram.

Optionally, the medical instrument further comprises a first pair of electrodes and a second pair of electrodes, the first pair of electrodes being used for sensing, the second pair of electrodes being used for sensing and stimulation, the second pair of electrodes comprising a tip electrode, the tip electrode being configured on the ventricular electrode lead and arranged at a myocardial position of a ventricle; the control device is configured to acquire the far-field electrocardiogram based on the first pair of electrodes, and to acquire the *in vivo* near-field myocardial electrocardiogram based on the second pair of electrodes.

Optionally, the second pair of electrodes are both configured on the ventricular electrode lead, and the medical instrument is used for providing a cardiac pacing function and/or a defibrillation therapy function via the ventricular electrode lead; or,
the first pair of electrodes are both configured on the ventricular electrode lead or configured as additional electrodes disposed within a blood vessel, within a cardiac chamber, on an epicardium, within a thoracic cavity other than heart, or subcutaneously, and the far-field electrocardiogram is an *in vivo* far-field myocardial electrocardiogram; or, the first pair of electrodes are configured as surface electrodes for application on the skin, and the far-field electrocardiogram is a surface electrocardiogram.

Optionally, the step of determining whether to deliver the CCM pulse stimulation to the myocardial position according to the R wave in the far-field electrocardiogram and the R wave in the *in vivo* near-field myocardial electrocardiogram specifically comprises:
acquiring a first sensing time of the R wave in the far-field electrocardiogram and a second sensing time of the R wave in the *in vivo* near-field myocardial electrocardiogram corresponding to the R wave in the far-field electrocardiogram, as well as a deliverable pulse time window corresponding to the R wave in the *in vivo* near-field myocardial electrocardiogram, wherein the deliverable pulse time window is a CCM stimulation safety window;
determining a pulse delivery start time according to the first sensing time or the second sensing time;
determining whether to deliver the CCM pulse stimulation to the myocardial position according to the pulse delivery start time and the deliverable pulse time window.

Optionally, the step of determining the pulse delivery start time according to the first sensing time or the second sensing time specifically comprises:
acquiring the pulse delivery start time according to the second sensing time and a second preset duration using the second sensing time as a reference zero point, or calculating a time difference between the second sensing time and the first sensing time; calculating a pulse delivery time according to the first sensing time, the time difference, and a third preset duration using the first sensing time as a reference zero point, wherein the third preset duration is equal to the second preset duration.

Optionally, the pulse stimulation control method further comprises a set-up period and an operational period; during the set-up period, calculating a first preset duration according to a difference between the first sensing time and the second sensing time and a third preset duration; and during the operational period, acquiring the pulse delivery start time according to the first sensing time and the first preset duration using the first sensing time as a reference zero point.

Optionally, the second preset duration is greater than or equal to 15 ms and less than or equal to 80 ms.

Optionally, the step of acquiring the first sensing time of the R wave in the far-field electrocardiogram and the second sensing time of the R wave in the *in vivo* near-field myocardial electrocardiogram corresponding to the R wave in the far-field electrocardiogram specifically comprises:
acquiring a first sensing event in the far-field electrocardiogram and a second sensing event in the far-field electrocardiogram, wherein at least one of the first sensing event and the second sensing event is an R wave;
confirming that the first sensing event in the far-field electrocardiogram corresponds to the second sensing event in the *in vivo* near-field myocardial electrocardiogram, and that both the first sensing event and the second sensing event are R waves when an absolute value of a difference between the first sensing time of the first sensing event in the far-field electrocardiogram and the second sensing time of the second sensing event in the *in vivo* near-field myocardial electrocardiogram is within a preset range; when the first sensing event in the far-field electrocardiogram is generated by ventricular activation originating from atrial conduction, the preset range is greater than or equal to 0 ms and less than or equal to 120 ms; when the R wave in the far-field electrocardiogram is generated by ventricular activation originating from a ventricle or by a ventricular pacing pulse, the preset range is greater than or equal to 0 ms and less than or equal to 250 ms.

Optionally, the step of acquiring the first sensing time of the R wave in the far-field electrocardiogram and the second sensing time of the R wave in the *in vivo* near-field myocardial electrocardiogram corresponding to the R wave in the far-field electrocardiogram specifically further comprises:
taking the first sensing time as a first time point, and taking a time point from corresponding first set duration before the first time point as a time reference zero point, acquiring the second sensing event in the *in vivo* near-field myocardial electrocardiogram after the time reference zero point, the first set duration being greater than or equal to 10 ms and less than or equal to 120 ms;
   or,
taking the second sensing time as a second time point, and taking a time point from corresponding second set duration before the second time point as a time reference zero point, acquiring the first sensing event in the far-field electrocardiogram after the time reference zero point, the set duration being greater than or equal to 30 ms and less than or equal to 120 ms.

Optionally, the step of acquiring the first sensing time of the R wave in the far-field electrocardiogram and the second sensing time of the R wave in the *in vivo* near-field myocardial electrocardiogram corresponding to the R wave in the far-field electrocardiogram further comprises:
acquiring a sensing time window corresponding to the first sensing event in the far-field electrocardiogram;
judging whether the second sensing time or the first sensing time falls within the sensing time window;
if yes, determining that the first sensing event in the far-field electrocardiogram corresponds to the second sensing event in the *in vivo* near-field myocardial electrocardiogram, and that both the first sensing event and the second sensing event are R waves; if not, determining that the first sensing event in the far-field electrocardiogram does not correspond to the second sensing event in the *in vivo* near-field myocardial electrocardiogram, and that one of the first sensing event and the second sensing event is not an R wave, and the control device being configured not to deliver the CCM pulse stimulation to the myocardial position.

Optionally, when the first sensing time is taken as the first time point, and the first sensing event is an R wave, the step of judging whether the second sensing time or the first sensing time falls within the sensing time window comprises: judging whether the second sensing time falls within the sensing time window;
when the second sensing time is taken as the second time point, and the second sensing event is an R wave, the step of judging whether the second sensing time or the first sensing time falls within the sensing time window comprises: judging whether the first sensing time falls within the sensing time window.

Optionally, when the first sensing event in the far-field electrocardiogram is ventricular activation which does not originate from ventricular pacing, if the first sensing time is earlier than the second sensing time, a starting point of the sensing time window corresponding to the first sensing event in the far-field electrocardiogram is determined based on the first sensing time; if the second sensing time is earlier than the first sensing time, the starting point of the sensing time window corresponding to the first sensing event in the far-field electrocardiogram is determined based on the second sensing time;
when the first sensing event in the far-field electrocardiogram is generated by the ventricular pacing pulse, the starting point of the sensing time window corresponding to the first sensing event in the far-field electrocardiogram is determined based on a delivery time of the pacing pulse to the ventricle, and the delivery time of the pacing pulse is considered as the first sensing time.

Optionally, the sensing time window has a start time earlier than or equal to the first sensing time or the second sensing time by a fourth preset duration, and the deliverable pulse time window has a second preset length; the sensing time window has a first preset length, and the second preset length is greater than the first preset length; the fourth preset duration is greater than or equal to 0 ms and less than or equal to 50 ms.

Optionally, the sensing time window has a first preset length, and when the R wave in the far-field electrocardiogram is generated by a heartbeat resulting from ventricular activation originating from atrial conduction, the first preset length has a range of greater than or equal to 60 m and less than 120 ms; when the R wave in the far-field electrocardiogram is generated by ventricular activation originating from the ventricle or by the ventricular pacing pulse, the first preset length is greater than or equal to 160 m and less than or equal to 250 ms; or, the first preset length is determined through program control.

Optionally, the number of the ventricular electrode leads is multiple and the ventricular electrode leads are arranged at multiple different ventricular myocardial positions, and the number of the *in vivo* near-field myocardial electrocardiograms and the second sensing events is multiple; the control device is further configured to execute the following steps:
presetting set sensing parameters corresponding to R waves at different myocardial positions, wherein the set sensing parameter comprises a set sensing time and/or a set sensing occurrence sequence;
setting all other remaining second sensing events as R waves when a first second sensing event falling within the sensing time window is an R wave; or,
setting all other remaining second sensing events as R waves when a last second sensing event falling within the sensing time window is an R wave.

Optionally, the step of determining whether to deliver the CCM pulse stimulation to the myocardial position according to the pulse delivery start time and the deliverable pulse time window specifically comprises:
delivering the CCM pulse stimulation to the myocardial position if the pulse delivery start time falls within the deliverable pulse time window; and if not, not delivering the CCM pulse stimulation to the myocardial position.

Optionally, the number of the ventricular electrode leads is multiple and the ventricular electrode leads are arranged at multiple different myocardial positions, and the number of the *in vivo* near-field myocardial electrocardiograms and the second sensing events is multiple; the control device is further configured to execute the following steps:
setting a pulse stimulation delivery sequence corresponding to the R waves at different myocardial positions, and delivering the CCM pulse stimulation to the different myocardial positions according to the pulse stimulation delivery sequence.

Optionally, when the R wave in the far-field electrocardiogram is ventricular activation which does not originate from ventricular pacing, a starting point of the deliverable pulse time window is determined based on the first sensing time of the R wave in the far-field electrocardiogram, or, based on the second sensing time, determined according to the first sensing time, of the corresponding R wave in the *in vivo* near-field myocardial electrocardiogram;
when the R wave in the far-field electrocardiogram is generated by a ventricular pacing pulse, the starting point of the deliverable pulse time window is determined based on a delivery time of the pacing pulse to the ventricle, and the delivery time of the pacing pulse is considered as the first sensing time.

Optionally, the deliverable pulse time window has a second preset length, the second preset length has a range of greater than or equal to 150 ms and less than or equal to 300 ms, or the second preset length is determined through program control; the starting point of the deliverable pulse time window is earlier than the first sensing time or the second sensing time by a fourth preset duration, and the fourth preset duration is greater than or equal to 0 ms and less than or equal to 50 ms.

Optionally, the control device is further configured to execute the following steps:
when the second sensing time is earlier than the first sensing time, determining the starting point of the deliverable pulse time window corresponding to the R wave in the *in vivo* near-field myocardial electrocardiogram according to the second sensing time, and determining the pulse delivery start time according to the first sensing time or the second sensing time;
when the second sensing time is later than the first sensing time, determining the starting point of the deliverable pulse time window corresponding to the R wave in the *in vivo* near-field myocardial electrocardiogram according to the first sensing time, and determining the pulse delivery start time according to the first sensing time or the second sensing time.

Optionally, the step of determining whether to deliver the CCM pulse stimulation to the myocardial position according to the pulse delivery start time and the deliverable pulse time window specifically comprises:
determining a pulse delivery stop time according to the pulse delivery start time;
delivering the CCM pulse stimulation to the myocardial position if both the pulse delivery start time and the pulse delivery stop time fall within the deliverable pulse time window, and if not, not delivering the CCM pulse stimulation to the myocardial position.

Optionally, the step of determining whether to deliver the CCM pulse stimulation to the myocardial position according to the pulse delivery start time and the deliverable pulse time window specifically comprises:
determining a pulse delivery stop time according to the pulse delivery start time and a preset pulse parameter;
redetermining the pulse parameter if the pulse delivery start time falls within the deliverable pulse time window, and the pulse delivery stop time does not fall within the deliverable pulse time window, so that both the pulse delivery start time and the pulse delivery stop time fall within the deliverable pulse time window, and delivering the CCM pulse stimulation to the myocardial position according to the redetermined pulse parameter;
not delivering the CCM pulse stimulation to the myocardial position if the pulse delivery start time does not fall within the deliverable pulse time window.

Optionally, the step of determining whether to deliver the CCM pulse stimulation to the myocardial position according to the pulse delivery start time and the deliverable pulse time window specifically comprises:
determining a pulse delivery stop time according to the pulse delivery start time and a preset pulse parameter if the pulse delivery start time falls within the deliverable pulse time window;
judging whether the pulse delivery stop time falls within the deliverable pulse time window;
if yes, delivering the CCM pulse stimulation;
if not, not delivering the CCM pulse stimulation, or, redetermining the pulse parameter, so that the pulse delivery stop time falls within the deliverable pulse time window; and delivering the CCM pulse stimulation according to the redetermined pulse parameter.

Optionally, the control device is configured to execute the following steps:
judging whether the ventricle has been captured if it is determined that a pacing pulse has been delivered to the myocardial position;
if yes, determining whether to deliver the CCM pulse stimulation to the myocardial position according to the pulse delivery start time and the deliverable pulse time window, wherein the deliverable pulse time window corresponding to the R wave in the *in vivo* near-field myocardial electrocardiogram is determined based on a delivery time of the pacing pulse to the ventricle;
if not, determining whether to deliver the CCM pulse stimulation to the myocardial position according to the pulse delivery start time and the deliverable pulse time window, wherein the deliverable pulse time window corresponding to the R wave in the *in vivo* near-field myocardial electrocardiogram is determined based on the first sensing time of the R wave in the far-field electrocardiogram, or, based on the second sensing time of the R wave in the *in vivo* near-field myocardial electrocardiogram.

Optionally, the step of judging whether the ventricle has been captured specifically comprises: judging whether the ventricle has been captured based on the far-field electrocardiogram.

Optionally, the step of delivering the CCM pulse stimulation to the myocardial position specifically comprises:
delivering the CCM pulse stimulation to the myocardial position if a current heart rate parameter is detected to be within a preset range.

Optionally, the CCM pulse stimulation is delivered during at least one of the following ventricular electrical activities:
a sinus beat, a ventricular beat originating from atrial conduction, a ventricular beat originating from a ventricle, and a ventricular beat originating from ventricular pacing.

On the basis of the common knowledge in the art, the optional conditions can be combined arbitrarily to obtain various embodiments of the present disclosure.

The positive and progressive effects of the present disclosure are as follows:
The CCM pulse stimulation is only delivered during the ventricular deliverable period: By confirming that the CCM pulse stimulation is delivered within the deliverable period corresponding to the overall ventricular electrical activity (R wave) reflected in the far-field electrocardiogram, the timeliness, safety, and effectiveness of the CCM pulse stimulation to the heart of a patient are guaranteed. Herein, to ensure the safety of the CCM pulse stimulation delivery time, using the far-field electrocardiogram to obtain the deliverable period information of ventricular electrical activity also represents a positive improvement in CCM pulse stimulation technology, further ensuring the safety, effectiveness, and therapeutic effect of CCM pulse stimulation for the patient.

Pulse stimulation is delivered only after the R wave: it is possible to timely analyze and process the sensing event in the far-field electrocardiogram or *in vivo* near-field electrocardiogram. False sensing events are automatically and accurately identified and excluded, and they are also determined as interference signals such as the T-wave instead of the R-wave signal. In this case, the CCM pulse stimulation is not delivered to the corresponding myocardial position, ensuring that the CCM pulse stimulation is not delivered under incorrect conditions, effectively reducing or avoiding the risk of inducing VT or VF, thereby preventing unnecessary pain or even safety hazards to patients. This ensures patient safety and enhances the reliability of pulse stimulation control; additionally, it ensures that the CCM pulse stimulation is delivered timely when the sensing event is confirmed as an R wave, that is, the CCM pulse stimulation is delivered only under correct conditions.

The *in vivo* far-field myocardial electrocardiogram is acquired through the first pair of electrodes configured on at least one ventricular electrode lead in the medical instrument, and the *in vivo* near-field myocardial electrocardiogram is acquired through the second pair of electrodes configured on at least one ventricular electrode lead in the medical instrument. The R wave in the *in vivo* far-field myocardial electrocardiogram is combined with the R wave in the *in vivo* near-field myocardial electrocardiogram to determine whether to deliver the CCM pulse stimulation to the myocardial position, thereby implementing the CCM therapy function.

Furthermore, compared to existing medical instruments, the use of the at least one ventricular electrode lead allows the implementation of not only the CCM therapy function but also the functions of existing traditional instruments (including cardiac pacing function and/or defibrillation therapy function). Therefore, the medical instrument provided by the present disclosure can provide both ICD and CCM therapies. For patients requiring SCD prevention and heart failure therapy, only the medical instrument provided by the present disclosure needs to be implanted in the patients, eliminating the need to implant two medical instruments.

### BRIEF DESCRIPTION OF THE DRAWINGS

FIG. 1 is a schematic diagram of configuring a first pair of electrodes and a second pair of electrodes on a right ventricular electrode lead in a medical instrument provided by an embodiment of the present disclosure.
FIG. 2 is another schematic diagram of configuring a first pair of electrodes and a second pair of electrodes on a right ventricular electrode lead in a medical instrument provided by an embodiment of the present disclosure.
FIG. 3 is yet another schematic diagram of configuring a first pair of electrodes and a second pair of electrodes on a right ventricular electrode lead in a medical instrument provided by an embodiment of the present disclosure.
FIG. 4 is a flow chart of a control method for a medical instrument provided by an embodiment of the present disclosure.
FIG. 5 is a flow chart of step S2 provided by an embodiment of the present disclosure.
FIG. 6 is a flow chart of step S3 provided by an embodiment of the present disclosure.
FIG. 7 is another flow chart of step S3 provided by an embodiment of the present disclosure.
FIG. 8 is yet another flow chart of step S3 provided by an embodiment of the present disclosure.
FIG. 9 is a partial flow chart of the control method for the medical instrument provided by an embodiment of the present disclosure.
FIG. 10 is another flow chart of step S2 provided by an embodiment of the present disclosure.
FIG. 11 is a schematic electrocardiogram corresponding to R-wave sensing according to an embodiment of the present disclosure.
FIG. 12 is another schematic electrocardiogram corresponding to R-wave sensing according to an embodiment of the present disclosure.
FIG. 13 is yet another schematic electrocardiogram corresponding to R-wave sensing according to an embodiment of the present disclosure.
FIG. 14 is still another schematic electrocardiogram corresponding to R-wave sensing according to an embodiment of the present disclosure.
FIG. 15 is a flow chart of steps S7 and S8 provided by an embodiment of the present disclosure.
FIG. 16 is a flow chart of steps S9 to S11 provided by an embodiment of the present disclosure.
FIG. 17 is a schematic diagram of steps S1003 and S1004 provided by an embodiment of the present disclosure.
FIG. 18 is a flow chart of steps S1005 to S1008 provided by an embodiment of the present disclosure.

### DETAILED DESCRIPTION OF THE PREFERRED EMBODIMENT

The following further illustrates the present disclosure through embodiments, but the present disclosure is not limited thereby to the scope of the disclosed embodiments.

It should be understood that the term "comprise" and its variations used in the present disclosure are open-ended inclusion, that is, "including but not limited to". The term "according to" refers to "at least partially according to", and the term "multiple" refers to "two or more".

It should be understood that although the terms "first", "second", "third", "fourth", etc. may be used in the present disclosure to describe various elements, these elements are not limited by these terms, and these terms are only used to distinguish one element from another.

### Embodiment 1

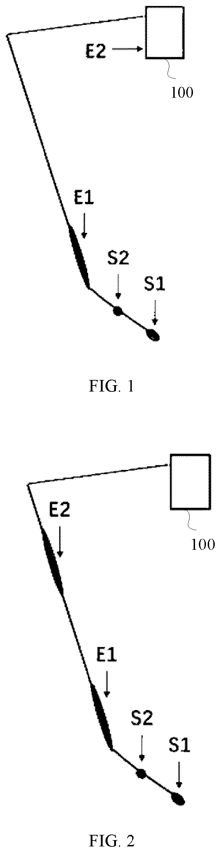

FIG. 1 is a schematic flow chart of a control method for a medical instrument provided in this embodiment. The control method may be executed by a control device of the medical instrument for delivering pulse stimulation. The control device may be implemented through software and/or hardware and can be a part of the medical instrument.

The medical instrument for delivering pulse stimulation in this embodiment includes at least one ventricular electrode lead and a control device. The at least one ventricular electrode lead is configured with a first pair of electrodes for sensing and a second pair of electrodes for sensing and stimulation, with at least one electrode of the second pair of electrodes arranged at a myocardial position of a ventricle. Herein, the first pair of electrodes and the second pair of electrodes may be configured on the same ventricular electrode lead or on different ventricular electrode leads.

In the medical instrument for delivering pulse stimulation provided in this embodiment, CCM therapy function can be provided by delivering CCM pulse stimulation to the myocardial position. Herein, the CCM pulse stimulation refers to pulse stimulation used for cardiac contractility modulation.

Optionally, the medical instrument is used for providing a cardiac pacing function and/or a defibrillation therapy function through the at least one ventricular electrode lead. In a specific implementation, the above medical instrument may be a single-chamber ICD or a dual-chamber ICD. Through its right ventricular electrode lead, it can not only achieve the cardiac pacing function and/or defibrillation therapy function, but also achieve the CCM therapy function. The above medical instrument may also be a CRT-D (cardiac resynchronization therapy defibrillator). Through its right ventricular electrode lead or left ventricular electrode lead, it can not only achieve the cardiac pacing function and/or defibrillation therapy function, but also achieve the CCM therapy function. The above medical instrument may also be a CRT-P (cardiac resynchronization therapy pacemaker), which can not only achieve the cardiac pacing function by means of its right ventricular electrode lead and/or left ventricular electrode lead, but also achieve the CCM therapy function by configuring a defibrillation electrode on its right ventricular electrode lead to acquire an *in vivo* far-field myocardial electrocardiogram. In a specific implementation, since the right ventricular electrode lead in a traditional ICD is configured with a pair of pacing electrodes and at least one defibrillation electrode, single-chamber pacemakers, dual-chamber pacemakers, and CRT-P can use the right ventricular electrode lead in the ICD to achieve the CCM therapy function. With the above configuration, the right ventricular electrode lead of CRT instruments (including CRT-P and CRT-D) can provide the CCM therapy function in the right ventricle in addition to pacing therapy.

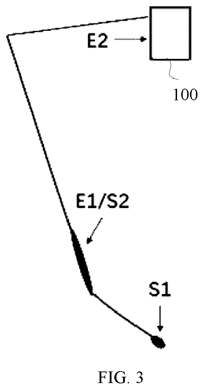

FIG. 1 to FIG. 3 respectively show schematic diagrams of configuring a first pair of electrodes and a second pair of electrodes on a lead in the right ventricle (RV) in the medical instrument. As shown in FIG. 1 to FIG. 3, the first pair of electrodes includes an electrode E1 and an electrode E2, which are used for sensing; the second pair of electrodes includes an electrode S1 and an electrode S2, which are used for sensing and stimulation. Specifically, as shown in FIG. 1, the electrode E1 is a spiral defibrillation electrode, the electrode S2 is a ring electrode, the electrode S1 is a tip electrode, and the electrode E2 is configured at the control device 100 of the medical instrument. As shown in FIG. 2, both the electrode E1 and the electrode E2 are spiral defibrillation electrodes, the electrode S2 is a ring electrode, and the electrode S1 is a tip electrode. As shown in FIG. 3, the electrode E2 is configured at the control device 100 of the medical instrument, the electrode E1 and the electrode S2 are spiral defibrillation electrodes, and the electrode S1 is a tip electrode. In FIG. 1 to FIG. 3, the electrode S1 is arranged at the myocardial position in the ventricle.

It should be noted that the specific configuration of the first pair of electrodes and the second pair of electrodes on the ventricular electrode lead is not limited to the forms shown in FIG. 1 to FIG. 3 and can be in other forms as well.

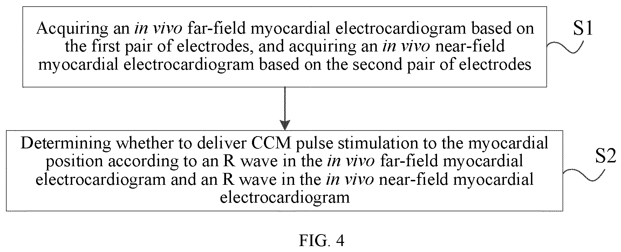

As shown in FIG. 4, the control method for the medical instrument provided in this embodiment may include steps S1 to S2 as follows:
Step S1: Acquiring an *in vivo* far-field myocardial electrocardiogram based on the first pair of electrodes, and acquiring an *in vivo* near-field myocardial electrocardiogram based on the second pair of electrodes.

Herein, the *in vivo* far-field myocardial electrocardiogram is also known as FF-EGM (Far-Field Electrogram), and the *in vivo* near-field myocardial electrocardiogram is also known as L-EGM (Local Electrogram, sometimes referred to as Near-field Electrogram).

Step S2: Determining whether to deliver CCM pulse stimulation to the myocardial position according to an R wave in the *in vivo* far-field myocardial electrocardiogram and an R wave in the *in vivo* near-field myocardial electrocardiogram.

It should be noted that the R wave in the *in vivo* far-field myocardial electrocardiogram and the R wave in the *in vivo* near-field myocardial electrocardiogram correspond to the same heartbeat.

In this embodiment, the *in vivo* far-field myocardial electrocardiogram is acquired through the first pair of electrodes configured on at least one ventricular electrode lead in the medical instrument, and the *in vivo* near-field myocardial electrocardiogram is acquired through the second pair of electrodes configured on at least one ventricular electrode lead in the medical instrument. The R wave in the *in vivo* far-field myocardial electrocardiogram is combined with the R wave in the *in vivo* near-field myocardial electrocardiogram to determine whether to deliver the CCM pulse stimulation to the myocardial position, thereby implementing the CCM therapy function.

Furthermore, compared to existing medical instruments, the use of the at least one ventricular electrode lead allows the implementation of not only the CCM therapy function but also the existing functions of traditional instruments (including cardiac pacing function and/or defibrillation therapy function). Therefore, the medical instrument provided in this embodiment can provide both ICD and CCM therapies. For patients requiring SCD prevention and heart failure therapy, only the medical instrument provided in this embodiment needs to be implanted in the patients, eliminating the need to implant two medical instruments.

Similarly, single-chamber pacemakers, dual-chamber pacemakers, and CRT-P using the right ventricular electrode lead from a traditional ICD can achieve the CCM therapy function while also providing the corresponding pacing therapy function for patients.

In a specific implementation, the above pulse stimulation is CCM stimulation, which may be delivered during at least one of the following ventricular electrical activities: a sinus beat, a ventricular beat originating from atrial conduction, a ventricular beat originating from a ventricle, and a ventricular beat originating from ventricular pacing. This represents an advancement over current CCM instruments, which only provide CCM pulse therapy for ventricular beats originating from atrial conduction.

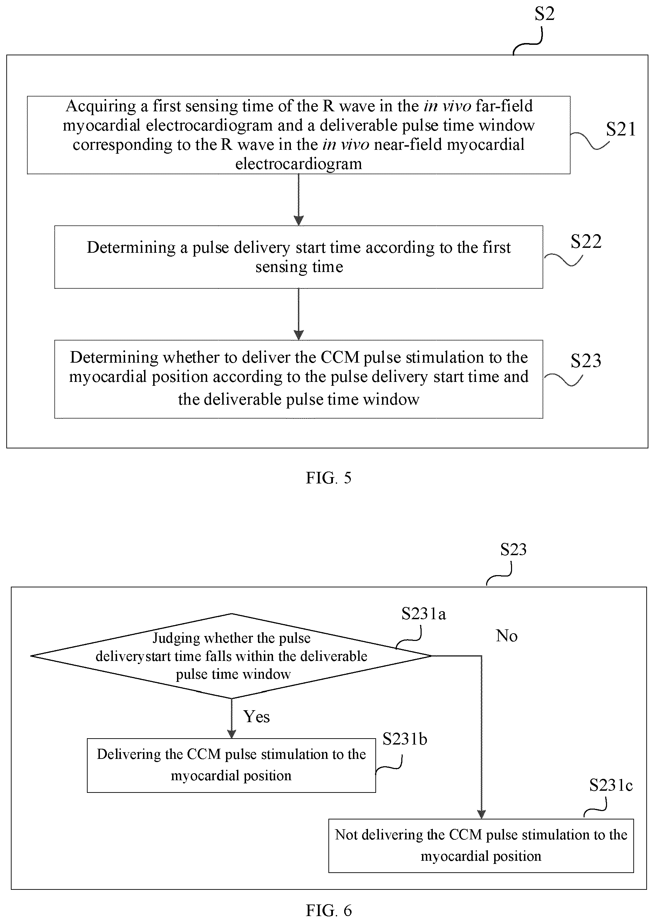

In an optional implementation, as shown in FIG. 5, the above step S2 includes steps S21 to S23 as follows:
S21: Acquiring a first sensing time of the R wave in the *in vivo* far-field myocardial electrocardiogram and a second sensing time of the R wave in the *in vivo* near-field myocardial electrocardiogram corresponding to the R wave in the far-field electrocardiogram, as well as a deliverable pulse time window corresponding to the R wave in the *in vivo* near-field myocardial electrocardiogram, wherein the deliverable pulse time window is a CCM stimulation safety window.

Herein, the first sensing time can be referred to as GS (Global Sense), and the deliverable pulse time window can be referred to as a CCM stimulation safety window (SSW). Herein, the deliverable pulse time window corresponds to a safety period, and the safety period corresponds to the CCM pulse deliverable period of the entire ventricle, with the goal of covering an absolute refractory period of the entire ventricular myocardium. Specifically, the deliverable pulse time window is determined according to the *in vivo* far-field myocardial electrocardiogram, with both its starting and ending points being adjustable. The starting point roughly corresponds to the "earliest" depolarization zone of the entire ventricular myocardium, while the ending point corresponds to or is slightly earlier than the end time point of the absolute refractory period of the ventricular myocardium.

In one example of a specific implementation, when the current R wave in the *in vivo* far-field myocardial electrocardiogram is generated by a heartbeat which does not originate from ventricular pacing (such as a spontaneous heartbeat), the starting point of the deliverable pulse time window can be determined according to the first sensing time GS.

Generally, the starting point of the deliverable pulse time window corresponding to the R wave in the *in vivo* near-field myocardial electrocardiogram is determined based on the first sensing time GS of the first sensing event in the *in vivo* far-field myocardial electrocardiogram, and thus the deliverable pulse time window can correspond to the CCM pulse deliverable period of the entire ventricle. The first sensing event is an R wave.

In special cases, the second sensing time of the second sensing event in the *in vivo* near-field myocardial electrocardiogram, corresponding to the first sensing event, can also be determined and can be referred to as LS (Local Sense), and then the starting point of the deliverable pulse time window is then determined according to the second sensing time LS. In such cases, there may be a delay in capturing the cardiac electrical signals, causing the first sensing time GS to lag behind the actual time point. Alternatively, for other reasons, the second sensing time LS of the second sensing event in the *in vivo* near-field myocardial electrocardiogram may be slightly earlier than the first sensing time GS of the first sensing event. In this case, by using the second sensing time LS as the starting time, the correspondence between local excitation events and overall excitation events can be more accurately determined. Therefore, the deliverable pulse time window corresponding to the R wave in the *in vivo* near-field myocardial electrocardiogram can also be determined based on the second sensing time LS of the R wave in the *in vivo* near-field myocardial electrocardiogram, and can also correspond to the CCM pulse deliverable period of the entire ventricle.

The deliverable pulse time window corresponding to the R wave in the *in vivo* near-field myocardial electrocardiogram can also be determined by the delivery time of the pacing pulse.

In another example of a specific implementation, when the R wave in the far-field electrocardiogram is generated by the ventricular pacing pulse, the starting point of the deliverable pulse time window is a delivery time of the pacing pulse. In this case, the delivery time of the pacing pulse is considered the first sensing time as referred to in various embodiments of the present disclosure.

Specifically, a second preset length of the deliverable pulse time window may be preset to 200 ms. The optional range includes but is not limited to 150 ms to 300 ms, as long as the end time point of the deliverable pulse time window does not exceed the absolute refractory period of the entire ventricular myocardium. The specific adjustment can be made adaptively according to the patient's condition (for example, whether antiarrhythmic drugs such as amiodarone are used, as antiarrhythmic drugs may prolong the absolute refractory period of the myocardium), the actual R wave sensing conditions (first sensing time GS and/or second sensing time LS), and other factors.

Step S22: Determining a pulse delivery start time according to the first sensing time GS or the second sensing time LS.

In an optional implementation of step S22, the pulse delivery start time is determined only according to the first sensing time GS. Specifically, the first sensing time GS may be used as a reference zero point, and the pulse delivery start time may be obtained by adding a first preset duration to the reference zero point. The first preset duration may be determined by calculating a time difference between the first sensing time GS and the second sensing time LS during the set-up period before the actual operational period. The reference zero point here represents the starting point of time.

In an optional implementation of step S22, the second sensing time LS corresponding to the second sensing event in the *in vivo* near-field myocardial electrocardiogram is determined according to the first sensing time GS; the pulse delivery start time is determined according to the second sensing time LS.

In a specific example, the pulse delivery start time is determined only according to the second sensing time. For example, the second sensing time may be used as the reference zero point, and the pulse delivery start time may be obtained by adding a second preset duration to the reference zero point.

In another specific example, the pulse delivery start time is determined according to both the first sensing time and the second sensing time. For example, the time difference between the second sensing time and the first sensing time may be calculated. Then, the first sensing time is used as the reference zero point, and the pulse delivery start time is obtained by adding the time difference and a third preset duration to the reference zero point.

The setting of the pulse delivery start time ensures that the pulse stimulation delivery time is within the absolute refractory period of the excitation event of the myocardium contacted by the electrode delivering the pulse.

Step S23: Determining whether to deliver the CCM pulse stimulation to the myocardial position according to the pulse delivery start time and the deliverable pulse time window.

In an optional implementation, whether to deliver the CCM pulse stimulation is determined according to whether the pulse delivery start time falls within the deliverable pulse time window. Specifically, as shown in FIG. 6, step S23 includes steps S231a to S231c as follows:
Step S231a: Judging whether the pulse delivery start time falls within the deliverable pulse time window; if yes, executing step S231b; if not, executing step S231c.

Step S231b: Delivering the CCM pulse stimulation to the myocardial position. Specifically, the CCM pulse stimulation begins be delivered to the myocardial position where the tip electrode S1 of the second pair of electrodes is located at the pulse delivery start time.

Step S231c: Not delivering the CCM pulse stimulation to the myocardial position.

In this implementation, the CCM pulse stimulation is delivered to the myocardial position only if the pulse delivery start time falls within the deliverable pulse time window, so that the CCM pulse stimulation must be delivered during the absolute refractory period of the entire ventricular myocardium, thereby ensuring the safety and reliability of the treatment for the patient.

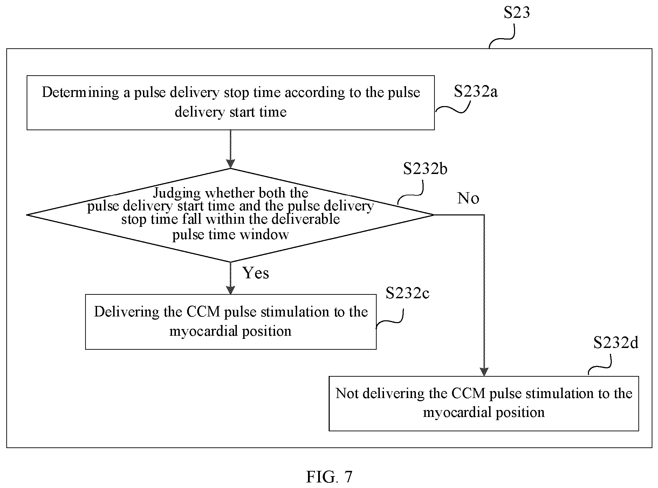

In another optional implementation, whether to deliver the CCM pulse stimulation is determined according to whether both the pulse delivery start time and the pulse delivery stop time fall within the deliverable pulse time window. Specifically, as shown in FIG. 7, step S23 includes steps S232a to S232d as follows:
Step S232a: Determining a pulse delivery stop time according to the pulse delivery start time.

Step S232b: Judging whether both the pulse delivery start time and the pulse delivery stop time fall within the deliverable pulse time window; if yes, executing step S232c; if not, executing step S232d.

Step S232c: Delivering the CCM pulse stimulation to the myocardial position. Specifically, the CCM pulse stimulation begins to be delivered to the myocardial position at the pulse delivery start time, and the CCM pulse stimulation stops being delivered to the myocardial position at the pulse delivery stop time.

Step S232d: Not delivering the CCM pulse stimulation to the myocardial position.

In this implementation, the CCM pulse stimulation is delivered to the myocardial position only if both the pulse delivery start time and the pulse delivery stop time fall within the deliverable pulse time window, so that all CCM pulse stimulation must be delivered during the absolute refractory period of the entire ventricular myocardium, thereby fully ensuring the safety and reliability of the treatment for the patient.

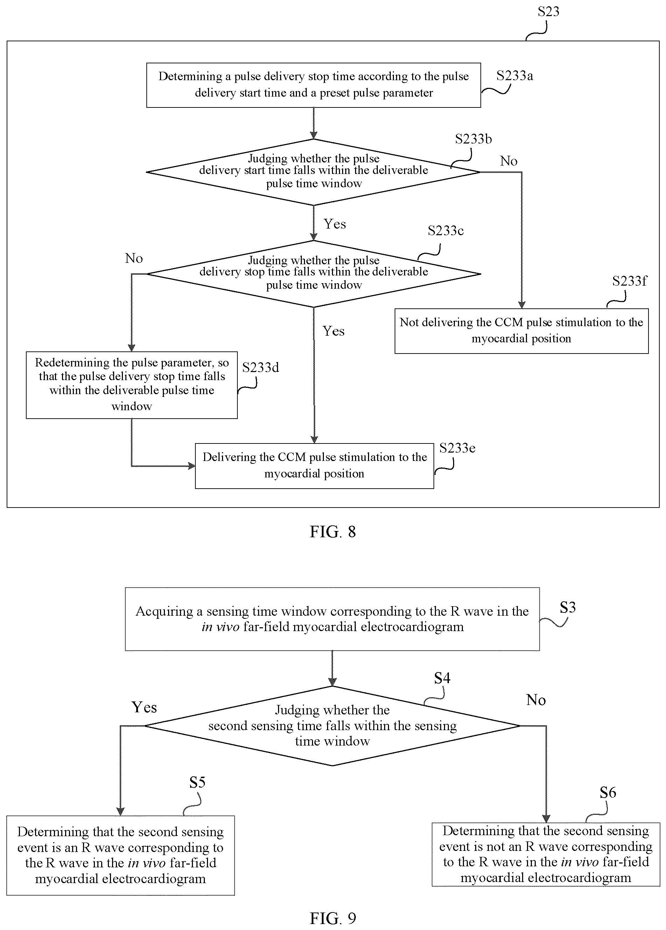

In yet another optional implementation of step S23, whether to deliver the CCM pulse stimulation is determined according to whether both the pulse delivery start time and the pulse delivery stop time fall within the deliverable pulse time window. Specifically, as shown in FIG. 8, step S23 includes steps S233a to S233d as follows:
Step S233a: Determining a pulse delivery stop time according to the pulse delivery start time and a preset pulse parameter. Herein, the pulse parameter may include the number of pulses delivered, pulse width, etc.

Step S233b: Judging whether the pulse delivery start time falls within the deliverable pulse time window; if yes, executing step S233c; if not, executing step S233f.

Step S233c: Judging whether the pulse delivery stop time falls within the deliverable pulse time window; if yes, executing step S233e; if not, executing step S233d.

Step S233d: Redetermining the pulse parameter, so that the pulse delivery stop time falls within the deliverable pulse time window. In a specific implementation, the pulse delivery stop time may fall within the deliverable pulse time window by reducing the number of pulses delivered, decreasing the pulse width, or by both reducing the number of pulses delivered and decreasing the pulse width.

Step S233e: Delivering the CCM pulse stimulation to the myocardial position. Specifically, the CCM pulse stimulation begins to be delivered to the myocardial position at the pulse delivery start time, and the CCM pulse stimulation stops being delivered to the myocardial position at the pulse delivery stop time.

In one example of a specific implementation, the pulse parameter needs to be redetermined if the pulse delivery start time falls within the deliverable pulse time window, and the pulse delivery stop time does not fall within the deliverable pulse time window, so that both the pulse delivery start time and the pulse delivery stop time fall within the deliverable pulse time window, and the CCM pulse stimulation is delivered to the myocardial position according to the redetermined pulse parameter.

In another example of a specific implementation, if both the pulse delivery start time and the pulse delivery stop time fall within the deliverable pulse time window, the CCM pulse stimulation is delivered to the myocardial position according to the preset pulse parameter.

Step S233f: Not delivering the CCM pulse stimulation to the myocardial position.

In this implementation, the pulse parameter is redetermined if the pulse delivery start time falls within the deliverable pulse time window, and the pulse delivery stop time does not fall within the deliverable pulse time window, so that the CCM pulse stimulation is delivered to the myocardial position only if both the pulse delivery start time and the pulse delivery stop time fall within the deliverable pulse time window, so that all CCM pulse stimulation must be delivered during the absolute refractory period of the entire ventricular myocardium, thereby fully ensuring the safety and reliability of the treatment for the patient, while also providing the maximum possible CCM therapy.

In yet another optional implementation, step S23 includes steps S234a to S234b as follows:
Step S234a: Determining a pulse delivery stop time if the pulse delivery start time falls within the deliverable pulse time window;

Step S234b: Determining whether to deliver the CCM pulse stimulation according to the pulse delivery stop time and the deliverable pulse time window.

In the medical instrument provided in this embodiment, when the pulse delivery start time falls within the deliverable pulse time window, the pulse delivery stop time is further determined. Finally, whether to deliver the CCM pulse stimulation is determined according to whether the pulse delivery stop time falls within the deliverable pulse time window. This effectively avoids the occurrence of delivering CCM pulse stimulation outside the absolute refractory period of the entire myocardium. Compared to existing cardiac contractility modulators, the medical instrument provided in this embodiment can improve the safety, effectiveness, and reliability of the treatment for the patient.

In an optional implementation of step S234b, it is judged whether the pulse delivery stop time falls within the deliverable pulse time window; if yes, the CCM pulse stimulation is delivered; if not, the CCM pulse stimulation is not delivered.

In another optional implementation, step S234a specifically includes: determining the pulse delivery stop time according to the pulse delivery start time and the preset pulse parameter; step S234b specifically includes: judging whether the pulse delivery stop time falls within the deliverable pulse time window; if yes, delivering the CCM pulse stimulation; if not, redetermining the pulse parameter, so that the pulse delivery stop time falls within the deliverable pulse time window; and delivering the CCM pulse stimulation according to the redetermined pulse parameter.

In an optional implementation, the step of delivering the CCM pulse stimulation to the myocardial position, such as the above steps S231b, S232c, and S233e, specifically includes: if it is determined that the second sensing event in the *in vivo* near-field myocardial electrocardiogram is an R wave corresponding to the R wave in the *in vivo* far-field myocardial electrocardiogram, then delivering the CCM pulse stimulation to the myocardial position.

In this implementation, by determining that the second sensing event in the *in vivo* near-field myocardial electrocardiogram is an R wave, it can be ensured that the CCM stimulation is triggered by the near-field R wave instead of other signals (such as T-wave, myoelectricity, or other non-myocardial depolarization electrical activity), effectively eliminating the occurrence of false triggering. Through the design of this critical step, the safety and effectiveness of CCM pulse stimulation can be effectively improved.

In an optional implementation, as shown in FIG. 9, the above control method also includes steps S3 to S6 as follows:
Step S3: Acquiring a sensing time window corresponding to the R wave in the *in vivo* far-field myocardial electrocardiogram.

Herein, the sensing time window can be referred to as the R-wave time window (RTW). Specifically, the sensing time window may be obtained according to the first sensing time and/or the second sensing time. First, the starting point of the sensing time window may be determined according to the first sensing time and/or the second sensing time. Then, the first preset length of the sensing time window may be determined according to the type of current ventricular activation. In a specific example, if the type of current activation is ventricular activation originating from atrial conduction, the width of the R wave in the *in vivo* far-field myocardial electrocardiogram is normal, typically greater than or equal to 60 ms and less than 120 ms. For example, the first preset length of the sensing time window may be determined as 100 ms. In another specific example, if the type of current activation is ventricular activation originating from the ventricle, the width of the R wave in the *in vivo* far-field myocardial electrocardiogram is greater than the normal width, typically between 160 ms and 250 ms. For example, the first preset length of the sensing time window may be determined as 200 ms. In yet another specific example, if the type of current activation is activation originating from ventricular pacing, the R wave in the *in vivo* far-field myocardial electrocardiogram is relatively wide, typically between 160 ms and 250 ms, and similarly, the first preset length of the sensing time window may be determined as 200 ms. Herein, the type of ventricular activation can be determined using conventional methods, such as detecting premature ventricular contractions (PVCs).

It should be noted that the first preset length of the sensing time window can also be obtained in other ways, such as being input by a doctor through an external device (e.g., a programmer) to modify the preset value and directly program the time window length. Herein, the starting points of the above deliverable pulse time window and the above sensing time window can be the same or different; the second preset length of the above deliverable pulse time window and the first preset length of the above sensing time window can be the same or different. In other words, the deliverable pulse time window and the sensing time window can be set in the same way or independently, without any correlation or interdependence. For example, different window lengths can be set, with different preset durations following the first sensing time and the second sensing time. Preferably, when the starting points of the deliverable pulse time window and the sensing time window are the same, the second preset length of the deliverable pulse time window is greater than the first preset length of the sensing time window.

Step S4: Judging whether the second sensing time falls within the sensing time window; if yes, executing step S5; if not, executing step S6.

Step S5: Determining that the second sensing event is an R wave corresponding to the R wave in the *in vivo* far-field myocardial electrocardiogram.

Step S6: Determining that the second sensing event is not an R wave corresponding to the R wave in the *in vivo* far-field myocardial electrocardiogram. Specifically, if the second sensing event is not an R wave corresponding to the R wave in the *in vivo* far-field myocardial electrocardiogram, it indicates that the second sensing event may be a T-wave or other interference signal.

In general, the R-wave sensing in the far-field electrocardiogram reflects the relatively early timing of ventricular electrical activity, and the second sensing time (LS) in the *in vivo* near-field myocardial electrocardiogram usually occurs after the first sensing time (GS). Therefore, the sensing time window is obtained based on the first sensing time (GS), and it is judged whether the second sensing time falls within the sensing time window. In special cases, due to possible sensing delays, the first sensing time (GS) may slightly lag behind the second sensing time (LS), and the difference between the two falls within a predetermined range (e.g., 20 ms to 120 ms). To more accurately determine the correspondence between the R wave in the far-field electrocardiogram and the second sensing event in the *in vivo* near-field myocardial electrocardiogram, the sensing time window can be obtained based on the second sensing time (LS). In this case, the second sensing time (LS) must fall within the sensing time window, and it can be directly confirmed that the second sensing event in the *in vivo* near-field myocardial electrocardiogram is an R wave corresponding to the R wave in the far-field electrocardiogram.

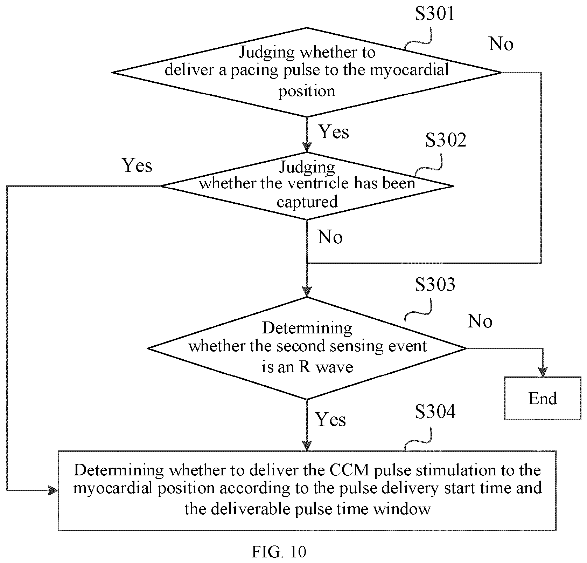

As shown in FIG. 10, the control method for the medical instrument provided in this embodiment may include steps S301 to S304 as follows:
Step S301: Judging whether to deliver a pacing pulse to the myocardial position; if yes, executing step S302; if not, executing step S303.

Step S302: Judging whether the ventricle has been captured; if not, executing step S303; if yes, executing step S304. In a specific implementation, there are several methods to judge whether the ventricle has been captured. For example, whether the ventricle has been captured can be judged based on the *in vivo* far-field myocardial electrocardiogram. It is certain that if the pacing pulse amplitude is sufficiently high and/or the pulse width is sufficiently long, the capture can be assumed to be certain without the need for specific determination, and step S304 can be executed directly.

Step S303: Determining whether the second sensing event in the *in vivo* near-field myocardial electrocardiogram is an R wave corresponding to the R wave in the *in vivo* far-field myocardial electrocardiogram; if yes, executing step S304; if not, ending the process.

Step 304: Determining whether to deliver the CCM pulse stimulation to the myocardial position according to the pulse delivery start time and the deliverable pulse time window. In a specific implementation, the first sensing time of the R wave in the *in vivo* far-field myocardial electrocardiogram and the deliverable pulse time window corresponding to the R wave in the *in vivo* near-field myocardial electrocardiogram are obtained, and the pulse delivery start time is determined according to the first sensing time. The step of determining whether to deliver the CCM pulse stimulation to the myocardial position according to the pulse delivery start time and the deliverable pulse time window is similar to the above step S23.

In this implementation, if it is determined to deliver a pacing pulse to the myocardial position, it indicates that the medical instrument is currently providing a pacing function, and it is necessary to further judge whether the ventricle has been captured. If it is judged that the ventricle has not been captured, it is necessary to further judge whether the second sensing event in the *in vivo* near-field myocardial electrocardiogram is a corresponding R wave. If it is an R wave, it is determined whether to deliver the CCM pulse stimulation to the myocardial position according to the pulse delivery start time and the deliverable pulse time window. If it is judged that the ventricle has been captured, then the pacing pulse time is taken as a pulse delivery start time, and it is directly determined whether to deliver the CCM pulse stimulation to the myocardial position according to the pulse delivery start time and the deliverable pulse time window.

In an optional implementation, the step of delivering the CCM pulse stimulation to the myocardial position, such as the above steps S231b, S232c, and S233e, specifically includes: delivering the CCM pulse stimulation to the myocardial position if a current heart rate parameter is detected to be within a preset range. Herein, if the current heart rate parameter is not within the preset range, it indicates that the current heart rate parameter of the patient is abnormal, and in this case, the CCM pulse stimulation is not delivered to the myocardial position.

Specifically, whether the current heart rate parameter is within the preset range can be judged by two adjacent R waves. The preset range can be set according to the actual situation of the patient, for example, it can be set to [40 bpm, 120 bpm]. If the current heart rate parameter is less than 40 bpm or greater than 120 bpm, it can be determined that the current heart rate parameter is abnormal.

In this implementation, the CCM pulse stimulation is only delivered to the myocardial position of the patient arranged with the second pair of electrodes when the current heart rate parameter is within the preset range, i.e., when the current heart rate parameter is normal, which can further ensure the safety and reliability of the treatment for the patient.

### Embodiment 2

Embodiment 2 is similar to Embodiment 1, and the common aspects will not be repeated here; the focus will be on the differences between the two. In this embodiment, the first pair of electrodes is no longer configured on the ventricular electrode lead, but instead is configured as additional electrodes disposed within a blood vessel, within a cardiac chamber, on an epicardium, within a thoracic cavity other than heart, or subcutaneously, e.g., subcutaneous defibrillation electrodes of a sub-Q ICD. These electrodes can also obtain the *in vivo* far-field myocardial electrocardiogram. It can be understood that the various methods described in Embodiment 1 are all applicable to the situation in Embodiment 2.

### Embodiment 3

Embodiment 3 is similar to Embodiment 1, and the common aspects will not be repeated here; the focus will be on the differences between the two. In this embodiment, the first pair of electrodes is no longer configured on the ventricular electrode lead, but instead is configured for attachment to the skin as a surface electrode, such as commonly used surface electrocardiogram electrodes, specially designed electrodes, or defibrillation electrodes for external defibrillators (e.g., AEDs). Thus, based on the first pair of electrodes, a surface electrocardiogram is acquired to replace the far-field myocardial electrocardiogram in Embodiment 1, and the first sensing time of the R wave in the surface electrocardiogram is acquired. The pulse delivery start time is determined according to the first sensing time, and it is judged whether the pulse delivery start time falls within the deliverable pulse time window. It can be understood that the various methods described in Embodiment 1 are all applicable to the situation in Embodiment 3.

In the present disclosure, the surface electrocardiogram and far-field myocardial electrocardiogram in Embodiments 1, 2, and 3 can be collectively referred to as far-field electrocardiogram. Those skilled in the art will appreciate that the R wave referred to in the present disclosure includes a QRS complex and a single R wave.

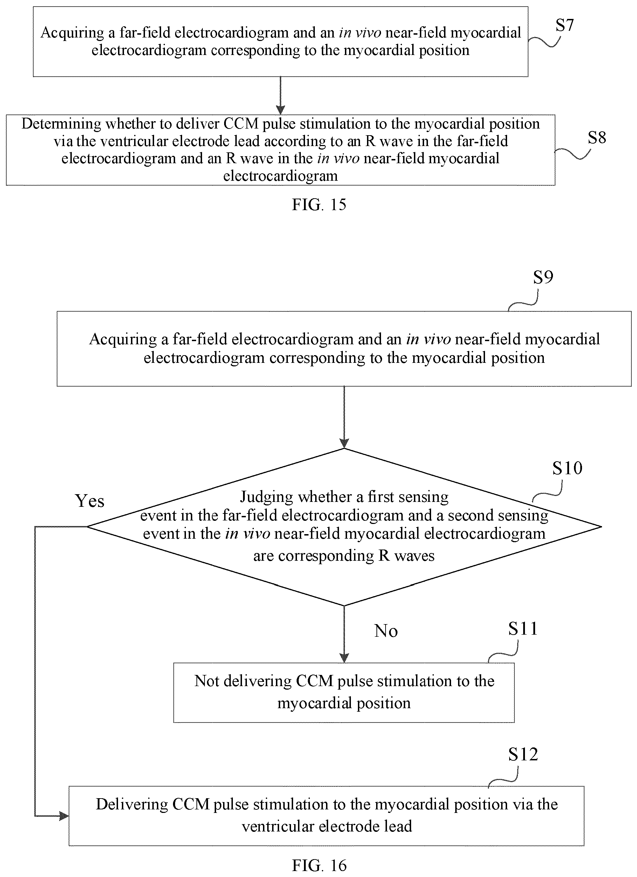

In summary, the present disclosure actually provides a medical instrument for delivering pulse stimulation, including at least one ventricular electrode lead and a control device, wherein the ventricular electrode lead is configured to be arranged at a myocardial position of a ventricle; the control device is configured to execute a pulse stimulation control method, as shown in FIG. 15, including the following steps:
Step S7: Acquiring a far-field electrocardiogram and an *in vivo* near-field myocardial electrocardiogram corresponding to the myocardial position;
Step S8: Determining whether to deliver CCM pulse stimulation to the myocardial position via the ventricular electrode lead according to an R wave in the far-field electrocardiogram and an R wave in the *in vivo* near-field myocardial electrocardiogram.

Using the schematic electrocardiogram corresponding to R-wave sensing as an example, the pulse stimulation control method in the embodiments of the present disclosure is further described in detail below.

Optionally, the first sensing time GS is taken as a first time point, and a time point from corresponding set duration before the first time point (programmable, ranging from, but not limited to, 10 ms to 200 ms, preferably, 30 ms to 120 ms, for example, 60 ms) is taken as a time reference zero point (starting point). Based on the time reference zero point, the second sensing time LS of the R wave in the *in vivo* near-field myocardial electrocardiogram is acquired.

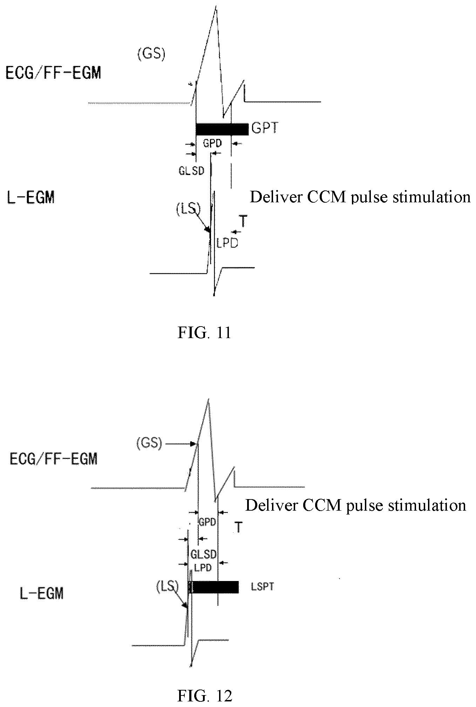

In an implementation, GPT (Global Pulse Time) is a deliverable pulse time window determined based on the first sensing time GS of the first sensing event in the far-field electrocardiogram (which can be either a surface electrocardiogram or an *in vivo* far-field myocardial electrocardiogram) and has a first preset length. The first sensing event is an R wave. As shown in FIG. 11, the control device first acquires the first sensing time GS of the R wave in the far-field electrocardiogram, generates the deliverable pulse time window GPT based on the first sensing time GS and the first preset length, and further acquires the second sensing time LS of the R wave in the near-field myocardial electrocardiogram corresponding to the R wave in the far-field electrocardiogram, with the second sensing time LS being later than the first sensing time GS. Using the second sensing time LS as the reference zero point, the pulse delivery start time T is obtained according to the second sensing time LS and a second preset duration LPD. Since the pulse delivery start time T falls within the deliverable pulse time window GPT, it is determined to deliver the CCM pulse stimulation to the second pair of electrodes (that is, after the second sensing time LS, waiting for the second preset duration until the pulse delivery start time T to deliver the CCM pulse stimulation).

The pulse delivery start time can also be determined based on the first sensing time GS. Specifically, using the first sensing time GS as the reference zero point, the pulse delivery start time T is obtained according to a difference GLSD between the second sensing time LS and the first sensing time GS (which is positive in this case) and a third preset duration (which is equal to the second preset duration LPD herein, and can range from 15 ms to 80 ms, generally 30 ms by default) (that is, after the first sensing time GS, waiting for the time length equal to the sum of the difference GLSD and the third preset duration until the pulse delivery start time T to deliver the CCM pulse stimulation).

In order to prevent false sensing of the R wave in the *in vivo* near-field myocardial electrocardiogram, such as mistaking the T wave for the R wave, the starting point of the sensing time window corresponding to the R wave in the far-field electrocardiogram can also be determined using the first sensing time GS as the reference zero point. The first preset length of the sensing time window can be equal to or shorter than the second preset length of the deliverable pulse time window GPT, and it is further judged whether the second sensing time LS falls within the sensing time window. If the second sensing time LS falls within the sensing time window, it is confirmed that the sensed event is the R wave in the *in vivo* near-field myocardial electrocardiogram corresponding to the R wave in the far-field electrocardiogram. If the second sensing time LS does not fall within the sensing time window, it is confirmed that the sensed event is not the R wave in the *in vivo* near-field myocardial electrocardiogram corresponding to the R wave in the far-field electrocardiogram, but rather another sensing event. In this case, the CCM pulse stimulation is not delivered.

In another implementation, as shown in FIG. 12, the control device first senses the second sensing time LS of the R wave in the *in vivo* near-field myocardial electrocardiogram, and then senses the first sensing time GS of the R wave in the far-field electrocardiogram. The occurrence of the second sensing time LS slightly earlier than the first sensing time GS may be due to a deviation caused by a delay in the R-wave sensing. In this case, the deliverable pulse time window LSPT is determined. LSPT (Local Sense Pulse Time) is a deliverable pulse time window determined based on the second sensing time LS of the R wave in the *in vivo* near-field myocardial electrocardiogram, with a preset window length. Further, using the second sensing time LS as the reference zero point, the pulse delivery start time T is obtained according to the second sensing time LS and the second preset duration LPD. At this time, the pulse delivery start time T falls within the LSPT, then it is determined to deliver the CCM pulse stimulation to the myocardial position where the second electrode is located (that is, after the second sensing time LS, waiting for the second preset duration until the pulse delivery start time T to deliver the CCM pulse stimulation).

Using the first sensing time GS as the reference zero point, the pulse delivery start time T may also be obtained according to a difference GLSD between the second sensing time LS and the first sensing time GS (which is negative in this case) and a third preset duration (which is equal to the second preset duration LPD herein) (that is, after the first sensing time GS, waiting for the time length equal to the sum of the difference GLSD and the third preset duration until the pulse delivery start time T to deliver the CCM pulse stimulation).

In order to prevent false sensing of the R wave in the *in vivo* near-field myocardial electrocardiogram, the starting point of the sensing time window corresponding to the R wave in the far-field electrocardiogram can also be determined using the second sensing time LS as the reference zero point. The first preset length of the sensing time window can be equal to or shorter than the second preset length of the deliverable pulse time window LSPT, and it is further judged whether the second sensing time LS falls within the sensing time window. Since the second sensing time LS must fall within the sensing time window at this time, it can be confirmed that the sensed event is the R wave in the *in vivo* near-field myocardial electrocardiogram corresponding to the R wave in the far-field electrocardiogram.

### Embodiment 4

For the above embodiments 1, 2, and 3, the pulse stimulation delivery method can be further divided into two phases: a set-up period and an operational period.

For a pulse stimulation system with an electrode at a single myocardial point, the following parameters need to be measured during the set-up period.

Referring to FIG. 11 or FIG. 12, the second sensing time LS is the first sensed second sensing event of the local electrocardiogram after the time reference zero point determined by the first sensing time GS.

GLSD is the difference between the second sensing time LS and the first sensing time GS; when the second sensing time LS is later than the first sensing time GS, the difference GLSD is a positive number, and when the second sensing time LS is earlier than the first sensing time GS, the difference GLSD is a negative number. The second preset duration LPD is the time length between the second sensing time LS and the pulse delivery start time T. As described in the previous embodiments, the time length GPD between the first sensing time GS and the pulse delivery start time T can be calculated based on the difference GLSD and the second preset duration LPD.

That is, GPD = GLSD + LPD.

During the operational period, the GPD calculated in the set-up period can be used as the first preset duration. Based on the first sensing time GS and the first preset duration GPD, CCM pulse stimulation is delivered to the corresponding myocardial position. At this time, after the R wave is sensed in the local myocardial electrocardiogram, the electrical stimulation output time can be directly determined based on the first preset duration GPD, without the need to calculate the pulse stimulation delivery time for the far-field myocardial electrocardiogram each time based on the first sensing time GS and the second sensing time LS, thus effectively reducing data processing time and improving the control efficiency of cardiac pulse stimulation triggering, while still achieving the desired stimulation effect.

In addition, the pulse delivery time (GPD) may be updated regularly or irregularly based on actual needs (where myocardial electrical stimulation may be continued or stopped). Then, myocardial electrical stimulation is continued according to the updated trigger time to achieve more flexible electrical stimulation effects and meet the requirements of various pulse electrical stimulation scenarios.

It should be noted that, preferably, the above parameters are averaged over several cardiac cycles (e.g., 6 cardiac cycles, which is programmable and may also be a different number of cycles); additionally, the set-up period should be performed separately during sinus electrical activity, ventricular ectopic activity, ventricular pacing, and the like.

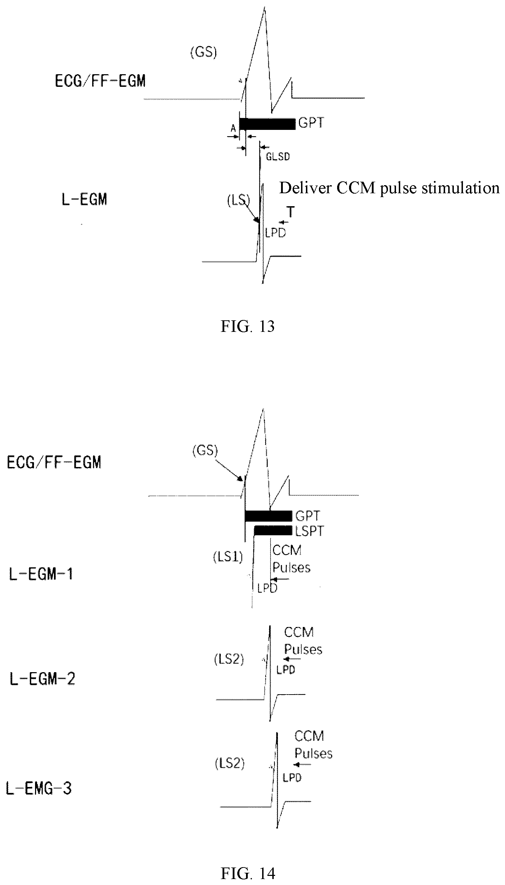

In another implementation, as shown in FIG. 13, the start time of the deliverable pulse time window GPT/LSPT may be different from the first sensing time GS or the second sensing time LS. Instead, it can be a moment that is earlier than the first sensing time GS or the second sensing time LS by a fourth preset duration A. By setting the fourth preset duration A, the errors caused by sensing delays can be compensated, thereby further improving the accuracy of R wave detection and the safety of pulse stimulation delivery.

Specifically, if the start time of the deliverable pulse time window GPT/LSPT is GPT-s/LSPT-s, then:
GPT-s = GS - A, where GLSD > 0 (i.e., LS is later than GS);
LSPT-s = GS + GLSD - A, where GLSD ≤ 0 (i.e., LS is earlier than or simultaneous with GS);
where 0 ms < A ≤ 50 ms, for example, the default value of A is 20 ms, and A is programmable and adjustable.

In other embodiments, the second sensing time LS may also be equal to the first sensing time GS, with the difference between the first sensing time and the second sensing time being 0 ms, i.e., A= 0 ms. In this case, either the first sensing time GS or the second sensing time LS can be used as the reference zero point, and the first preset time, the second preset time, and the third preset time are all equal.

### Embodiment 5

The difference between this embodiment and the above embodiments is that the medical instrument includes multiple ventricular electrode leads, each arranged at different myocardial positions, with multiple second electrodes in contact with the tissues at these myocardial positions. According to the control device described in Embodiments 1, 2, and 3, the CCM pulse stimulation is delivered to each myocardial position in a timely and effective manner, ensuring the safety, effectiveness, and reliability of the treatment for the patient.

Herein, the sensing time window is determined according to the first sensing time of the R wave in the far-field electrocardiogram and the second sensing time corresponding to the closest second sensing event.

For example, as shown in FIG. 14, an illustration is provided by presetting a pulse stimulation electrode at each of three different set myocardial positions (A, B, and C) in a patient, the set myocardial positions A, B, and C correspond to stimulation electrode pairs E1, E2, and E3 respectively, and the second sensing times of the corresponding second sensing events are LS1, LS2, and LS3 respectively, wherein the occurrence times of LS1, LS2, and LS3 are sequential (i.e., the first second sensing event occurs earliest, and is therefore closest to the first sensing time, and the other second sensing events occur sequentially in subsequent times).

Specifically, when acquiring, based on the electrode pair E1 at the set myocardium position A, the second sensing event from the *in vivo* near-field myocardial electrocardiogram corresponding to the myocardial position, the second sensing time LS1 corresponding to the second sensing event is acquired, and whether the second sensing time LS1 falls within the sensing time window corresponding to the R wave in the far-field electrocardiogram is determined; if the second sensing time does not fall within the window, it is determined that the second sensing event is not the R-wave signal corresponding to the R wave in the far-field electrocardiogram, but rather other interference signals such as T wave, and thus, the CCM pulse stimulation is not delivered to the stimulation electrode pair E1 at the set myocardial position A; if the second sensing time falls within the window, it is determined that the second sensing event is the local myocardial R-wave signal corresponding to the R wave in the far-field electrocardiogram, and then the pulse delivery time corresponding to the stimulation electrode corresponding to the set myocardial position A is timely and accurately calculated at the second sensing time corresponding to the second sensing event; next, it is determined whether the pulse delivery time falls within the deliverable pulse time window GPT corresponding to the R wave in the far-field electrocardiogram, and if yes, the pulse stimulation is controlled to be delivered to the stimulation electrode at the set myocardial position A at the pulse delivery time, otherwise, it is determined that no CCM pulse stimulation will be delivered to the stimulation electrode at the set myocardial position A, thus completing one pulse stimulation control for the set myocardial position A.

Similarly, the pulse stimulation control process for the set myocardial positions B and C is analogous to the pulse stimulation control process for the set myocardial position A, and therefore, the details are not described herein.

After each second sensing event is determined to be R-wave sensing, when the pulse delivery time calculated based on the second sensing time corresponding to the second sensing event falls within the deliverable pulse time window (GPT) corresponding to the R wave in the far-field electrocardiogram, the second sensing time (LS1) corresponding to the first second sensing event can also be used as a trigger point (reference zero point) for the pulse delivery time window (LSPT) to ensure that the pulse delivery time of subsequent LSn (n > 1, such as LS2 and LS3) falls within the LSPT window.

It should be noted that the pulse stimulation control processes for different set myocardial positions can be independent of each other and will not interfere with or affect each other. For example, during the pulse stimulation control process at the set myocardial position A, or after the completed pulse stimulation control at the set myocardial position A, as long as a second sensing event LS2 appears in the *in vivo* near-field myocardial electrocardiogram corresponding to the set myocardial position B, the aforementioned pulse stimulation control process can be independently executed, and the pulse stimulation control for all set myocardial positions will be ultimately completed in an orderly and efficient manner, thereby effectively guaranteeing the safety and reliability of CCM pulse stimulation for patients. Different pulse stimulation delivery sequences can also be set for different set myocardial positions. For example, during three consecutive time periods, the ventricular electrode leads respectively stimulate myocardial positions A, B, and C within their respective time periods. For another example, during one time period, pulse stimulation is only performed at the set myocardial position A, while during another time period, pulse stimulation is only performed at the set myocardial positions B and C, so as to balance the requirements for both safety and effectiveness.

In an implementation, when the sensing times corresponding to the second sensing events in multiple *in vivo* near-field myocardial electrocardiograms occur very close together (i.e., within a small time span), the pulse stimulation control method according to this embodiment further includes the following steps:
acquiring the second sensing time LS corresponding to the second sensing event in the *in vivo* near-field myocardial electrocardiogram corresponding to each *in vivo* myocardial electrocardiogram to identify the second sensing event which occurs earliest as the first second sensing event; acquiring the second sensing time LS 1 corresponding to the first second sensing event, and judging whether the second sensing time LS 1 falls within the sensing time window corresponding to the R wave in the far-field electrocardiogram, and if the second sensing time LS1 falls within the window, determining the second sensing event as the local myocardial R-wave signal corresponding to the R wave in the far-field electrocardiogram; and
for the second sensing events in the local myocardial electrocardiograms corresponding to the remaining myocardial positions, judging whether the second sensing times corresponding to these second sensing events fall within the aforementioned sensing time window, and if yes, directly determining that the second sensing events in the *in vivo* near-field myocardial electrocardiograms corresponding to the remaining myocardial positions are also R-wave signals corresponding to the R wave in the far-field electrocardiogram when the first second sensing event is determined as the R-wave signal corresponding to the R wave in the far-field electrocardiogram. In this case, there is no need for individual judgment and analysis of the second sensing events in the local myocardial electrocardiograms corresponding to the remaining myocardial positions, achieving accurate judgment while greatly simplifying the data analysis process, effectively reducing data processing time, and lowering the computational power requirements of devices. This further ensures the timeliness, accuracy, and effectiveness of the pulse stimulation control for patients.

It should be noted that the specific method used to determine whether the second sensing events in multiple local myocardial electrocardiograms are R waves may be selected based on the requirements of the actual scenario. A single execution scheme may be selected or multiple execution schemes may be combined to meet the higher demands of the cardiac electrical ventricular conduction scenario. This greatly enhances the practicality of pulse stimulation control and significantly improves the safety and effectiveness of the treatment for the patient.

Therefore, when there are multiple set myocardial positions, the pulse stimulation control method according to this embodiment further includes:
(1) presetting set sensing parameters corresponding to the R waves at different set myocardial positions;
   wherein the set sensing parameter includes the set sensing time and/or the set sensing occurrence sequence;
(2) setting the second sensing events in the other remaining local myocardial electrocardiograms all as R waves if the second sensing event in the *in vivo* near-field myocardial electrocardiogram that occurs first in the corresponding sensing time window is an R wave; or,
(3) setting the second sensing events in the other remaining local myocardial electrocardiograms all as R waves if the second sensing event in the *in vivo* near-field myocardial electrocardiogram that occurs last in the corresponding sensing time window is an R wave.

It should be noted that for single-electrode and multi-electrode pulse stimulation systems, the CCM pulse stimulation delivery time relative to each myocardial position is after the local myocardial sensing time, for example, after 40 ms.

### Embodiment 6

This embodiment is similar to Embodiment 1, and the common aspects will not be repeated here. The difference lies in the method of acquiring the first sensing time of the R wave in the far-field electrocardiogram and the second sensing time of the R wave in the *in vivo* near-field myocardial electrocardiogram corresponding to the R wave in the far-field electrocardiogram.

In this embodiment, the second sensing time of the second sensing event in the *in vivo* near-field myocardial electrocardiogram is used as a second time point, and the time point from corresponding second set duration before the second time point is taken as a time reference zero point. The first sensing event in the far-field electrocardiogram after the time reference zero point is then acquired. Optionally, the second set duration is greater than or equal to 10 ms and less than or equal to 120 ms. Herein, the second sensing event in the *in vivo* near-field myocardial electrocardiogram is an R wave. By identifying the first sensing event after the aforementioned method, it can be determined whether the first sensing event in the far-field electrocardiogram is an R wave corresponding to the R wave in the *in vivo* near-field myocardial electrocardiogram. It can be understood that whether the first sensing event or the second sensing event is acquired first, either can be used as the first time point to obtain the other corresponding thereto. By using the method of setting a sensing time window described in Embodiments 1 to 5, it can be determined whether the second sensing event in the *in vivo* near-field myocardial electrocardiogram corresponds to the first sensing event in the far-field electrocardiogram. As long as one of them is an R wave, this correspondence can be used to determine whether the other is also an R wave, thereby ensuring that CCM pulse stimulation is not delivered in the event of false sensing.

According to the above embodiments 1 to 6, it is known that the present disclosure also provides a medical instrument for delivering pulse stimulation, the medical instrument comprising at least one ventricular electrode lead and a control device, the ventricular electrode lead being configured to be arranged at a myocardial position of a ventricle; as shown in FIG. 16, the control device being configured to execute a pulse stimulation control method, comprising:
Step S9: Acquiring a far-field electrocardiogram and an *in vivo* near-field myocardial electrocardiogram corresponding to the myocardial position;
Step S10: Judging whether a first sensing event in the far-field electrocardiogram and a second sensing event in the *in vivo* near-field myocardial electrocardiogram are corresponding R waves; if not, executing step S11; if yes, executing step S12;
Step S11: Not delivering CCM pulse stimulation to the myocardial position;
Step S12: Delivering CCM pulse stimulation to the myocardial position via the ventricular electrode lead.

Herein, before executing step S12, the pulse delivery start time and/or pulse delivery stop time and whether the deliverable pulse time window falls within the deliverable pulse time window may also be acquired through the first sensing time of the first sensing event and the second sensing time of the second sensing event. It may also be determined whether the pulse delivery start time and/or pulse delivery stop time falls within the deliverable pulse time window. The details have been described in detail in Embodiment 1 and will not be repeated here.

It can be understood by those skilled in the art that, in the prior art, false sensing events may occur due to signal interference, leading to the sensing of events other than the R wave, such as the T wave. This significantly increases the risk of inducing malignant ventricular arrhythmias like VT (ventricular tachycardia) or VF (ventricular fibrillation), thereby increasing the patient's risk of mortality and subjecting the patient to additional painful stimulation. The pulse stimulation control method provided by the present disclosure can effectively identify such false sensing, thereby preventing the improper delivery of CCM pulse stimulation.

Optionally, step S10 further includes:
acquiring the first sensing time GS of the first sensing event in the far-field electrocardiogram and the second sensing time LS of the second sensing event in the *in vivo* near-field myocardial electrocardiogram; wherein at least one of the first sensing event and the second sensing event is an R-wave; and
confirming that the first sensing event in the far-field electrocardiogram corresponds to the second sensing event in the *in vivo* near-field myocardial electrocardiogram, and that both the first sensing event and the second sensing event are R waves when the absolute value of the difference between the first sensing time GS of the first sensing event in the far-field electrocardiogram and the second sensing time LS of the second sensing event in the *in vivo* near-field myocardial electrocardiogram is within a preset range; when the first sensing event in the far-field electrocardiogram is generated by ventricular activation originating from atrial conduction, the preset range is greater than or equal to 0 ms and less than or equal to 120 ms; when the R wave in the far-field electrocardiogram is generated by ventricular activation originating from a ventricle or by a ventricular pacing pulse, the preset range is greater than or equal to 0 ms and less than or equal to 250 ms; preferably, the preset range is 100 ms.

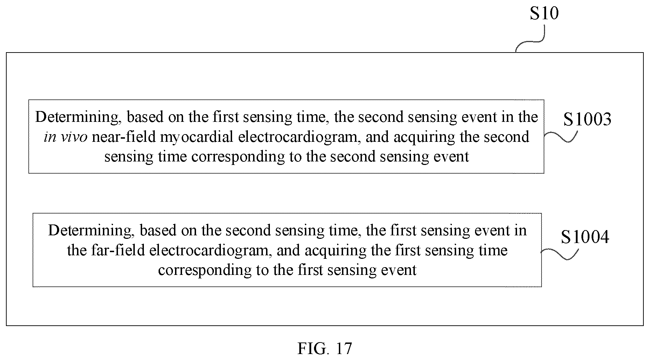

Optionally, as shown in FIG. 17, step S10 further includes:
Step S1003: Determining, based on the first sensing time, the second sensing event in the *in vivo* near-field myocardial electrocardiogram, and acquiring the second sensing time corresponding to the second sensing event, wherein the first sensing time is an R wave; or
Step S1004: Determining, based on the second sensing time, the first sensing event in the far-field electrocardiogram, and acquiring the first sensing time corresponding to the first sensing event, wherein the second sensing time is an R wave.

It can be understood that when acquiring the second sensing time based on the first sensing time, if a new first sensing time appears, the second sensing time has still not been acquired; or when acquiring the first sensing time based on the second sensing time, if a new second sensing time appears, the first sensing time has still not been acquired, then the control device is configured to restart a new round of judgment.

Optionally, step S1003 further includes: taking the first sensing time as a first time point, and taking a time point from corresponding first set duration before the first time point as a time reference zero point, acquiring the second sensing event in the *in vivo* near-field myocardial electrocardiogram after the time reference zero point, the first set duration being greater than or equal to 10 ms and less than or equal to 120 ms; or,

Optionally, step S1004 further includes: taking the second sensing time as a second time point, and taking a time point from corresponding second set duration before the second time point as a time reference zero point, acquiring the first sensing event in the far-field electrocardiogram after the time reference zero point, the second set duration being greater than or equal to 10 ms and less than or equal to 120 ms.

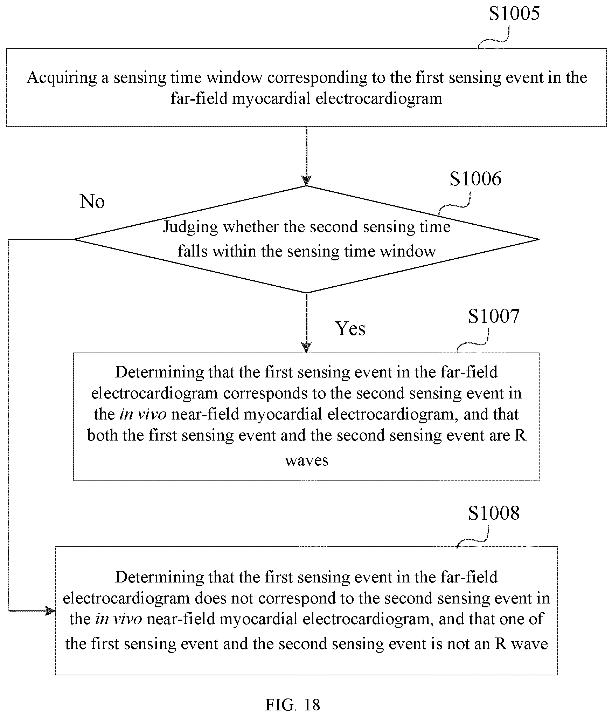

Optionally, as shown in FIG. 18, step S10 further includes:
Step S1005: Acquiring a sensing time window corresponding to the first sensing event in the far-field myocardial electrocardiogram;
Step S1006: Judging whether the second sensing time falls within the sensing time window; if yes, executing step S1007; if not, executing step S1008;
Step S1007: Determining that the first sensing event in the far-field electrocardiogram corresponds to the second sensing event in the *in vivo* near-field myocardial electrocardiogram, and that both the first sensing event and the second sensing event are R waves.

Step S1008: Determining that the first sensing event in the far-field electrocardiogram does not correspond to the second sensing event in the *in vivo* near-field myocardial electrocardiogram, and that one of the first sensing event and the second sensing event is not an R wave.

Step S1005 and step S1006 have been described in detail in Embodiments 1 to 7 and will not be repeated here.

In various embodiments of the present disclosure, the pulse delivery time window is a new requirement for CCM pulse stimulation delivery time, especially for the case of multiple stimulation sites with CCM pulse stimulation delivery. The delivery time of each stimulation site needs to fall within the pulse delivery time window, that is, within the deliverable period of the entire ventricle of the same heartbeat (ventricular excitation), not just the deliverable period of local ventricular myocardium at the electrode site.

The sensing time window, as a "global ventricular" or "far-field ventricular" R-wave sensing time window, is used to judge whether the acquired second sensing event is an R-wave sensing corresponding to local ventricular depolarization corresponding to the R wave in the far-field electrocardiogram (i.e., the "global ventricular" or *"in vivo* far-field" R-wave sensing corresponding to ventricular depolarization). The pulse deliverable time window (serving as a safety zone for stimulation delivery, corresponding to the deliverable period of the ventricular myocardium as a whole ("global ventricle" or "far-field ventricle")) is used to judge whether the pulse stimulation delivery time corresponding to the second sensing time LS is safe.

The pulse delivery time window and the sensing time window are independent parameters that can be separately controlled by a program to meet actual parameter configuration requirements. Moreover, to reduce the complexity of program control, doctors may select the same numerical value for both parameters (when appropriate). Alternatively, the system may directly assign the same numerical value to both parameters in advance, but the functionality of programmatically controlling the two parameters separately must be maintained. The two steps may be used together or separately.

In this embodiment, the CCM pulse stimulation is only delivered during the ventricular deliverable period: By confirming that the pulse stimulation delivery time falls within the pulse delivery window corresponding to the overall ventricular electrical activity (R wave) in the surface electrocardiogram or the *in vivo* far-field myocardial electrocardiogram, the timeliness, safety, and effectiveness of the CCM pulse stimulation to the heart of a patient are guaranteed. Herein, to ensure the safety of the pulse stimulation delivery time, using the surface electrocardiogram or the far-field myocardial electrocardiogram to obtain the deliverable period information of ventricular electrical activity also represents a positive improvement in pulse stimulation technology, further ensuring the safety, effectiveness, and therapeutic effect of CCM pulse stimulation for the patient.

In addition, the CCM pulse stimulation is delivered only after the R wave, it is possible to timely analyze and process the second sensing event in the *in vivo* near-field myocardial electrocardiogram. False sensing events are automatically and accurately identified and excluded, and they are also determined as interference signals such as the T-wave instead of the R-wave signal corresponding to the R wave in the far-field electrocardiogram. In this case, the CCM pulse stimulation is not delivered to the corresponding myocardial position, ensuring that the CCM pulse stimulation is not delivered under incorrect conditions, effectively avoiding the risk of inducing VT or VF, thereby preventing unnecessary pain or even safety hazards to patients. This ensures patient safety and enhances the reliability of pulse stimulation control; additionally, it ensures that the CCM pulse stimulation is delivered timely when the sensing event is confirmed as an R wave, that is, the CCM pulse stimulation is delivered only under correct conditions.

In a specific implementation, the medical instrument provided in this embodiment includes at least one processor and a memory in communication with the at least one processor. Herein, the memory stores a computer program that can be run by the at least one processor, and the computer program is executed by the at least one processor, so that the at least one processor can execute the control method provided in this embodiment. Herein, the processor corresponds to the control device described above.

It should be noted that the above control method may also be referred to as a pulse stimulation control method.

Although specific embodiments of the present disclosure have been described above, it should be understood by those skilled in the art that these embodiments are merely illustrative and that the protection scope of the present disclosure is defined by the appended claims. Various changes or modifications may be made to these embodiments by those skilled in the art without departing from the principle and spirit of the present disclosure, and such changes and modifications shall fall within the protection scope of the present disclosure.

## Claims

1. A medical instrument for delivering pulse stimulation, wherein the medical instrument comprises at least one ventricular electrode lead and a control device, the ventricular electrode lead being configured to be arranged at a myocardial position; the control device being configured to execute a pulse stimulation control method, the pulse stimulation control method comprising the following steps:
acquiring a far-field electrocardiogram and an *in vivo* near-field myocardial electrocardiogram corresponding to the myocardial position;
determining whether to deliver CCM pulse stimulation to the myocardial position via the ventricular electrode lead according to an R wave in the far-field electrocardiogram and an R wave in the *in vivo* near-field myocardial electrocardiogram.

2. The medical instrument according to claim 1, wherein the medical instrument further comprises a first pair of electrodes and a second pair of electrodes, the first pair of electrodes being used for sensing, the second pair of electrodes being used for sensing and stimulation, the second pair of electrodes comprising a tip electrode, the tip electrode being configured on the ventricular electrode lead and arranged at a myocardial position of a ventricle; the control device is configured to acquire the far-field electrocardiogram based on the first pair of electrodes, and to acquire the *in vivo* near-field myocardial electrocardiogram based on the second pair of electrodes.

3. The medical instrument according to claim 2, wherein the second pair of electrodes are both configured on the ventricular electrode lead, and the medical instrument is used for providing a cardiac pacing function and/or a defibrillation therapy function via the ventricular electrode lead; or,
the first pair of electrodes are both configured on the ventricular electrode lead or configured as additional electrodes disposed within a blood vessel, within a cardiac chamber, on an epicardium, within a thoracic cavity other than heart, or subcutaneously, and the far-field electrocardiogram is an *in vivo* far-field myocardial electrocardiogram; or, the first pair of electrodes are configured as surface electrodes for application on the skin, and the far-field electrocardiogram is a surface electrocardiogram.

4. The medical instrument according to claim 1, wherein the step of determining whether to deliver the CCM pulse stimulation to the myocardial position according to the R wave in the far-field electrocardiogram and the R wave in the *in vivo* near-field myocardial electrocardiogram specifically comprises:
acquiring a first sensing time of the R wave in the far-field electrocardiogram and a second sensing time of the R wave in the *in vivo* near-field myocardial electrocardiogram corresponding to the R wave in the far-field electrocardiogram, as well as a deliverable pulse time window corresponding to the R wave in the *in vivo* near-field myocardial electrocardiogram, wherein the deliverable pulse time window is a CCM stimulation safety window;
determining a pulse delivery start time according to the first sensing time or the second sensing time;
determining whether to deliver the CCM pulse stimulation to the myocardial position according to the pulse delivery start time and the deliverable pulse time window.

5. The medical instrument according to claim 4, wherein the step of determining the pulse delivery start time according to the first sensing time or the second sensing time specifically comprises:
acquiring the pulse delivery start time according to the second sensing time and a second preset duration using the second sensing time as a reference zero point; or calculating a time difference between the second sensing time and the first sensing time, calculating a pulse delivery time according to the first sensing time, the time difference, and a third preset duration using the first sensing time as a reference zero point, wherein the third preset duration is equal to the second preset duration.

6. The medical instrument according to claim 4, wherein the pulse stimulation control method further comprises setting a set-up period and an operational period; during the set-up period, calculating a first preset duration according to a difference between the first sensing time and the second sensing time and a third preset duration; and during the operational period, acquiring the pulse delivery start time according to the first sensing time and the first preset duration using the first sensing time as a reference zero point.

7. The medical instrument according to claim 5, wherein the second preset duration is greater than or equal to 15 ms and less than or equal to 80 ms.

8. The medical instrument according to claim 4, wherein the step of acquiring the first sensing time of the R wave in the far-field electrocardiogram and the second sensing time of the R wave in the *in vivo* near-field myocardial electrocardiogram corresponding to the R wave in the far-field electrocardiogram specifically comprises:
acquiring a first sensing event in the far-field electrocardiogram and a second sensing event in the far-field electrocardiogram, wherein at least one of the first sensing event and the second sensing event is an R wave;
confirming that the first sensing event in the far-field electrocardiogram corresponds to the second sensing event in the *in vivo* near-field myocardial electrocardiogram, and that both the first sensing event and the second sensing event are R waves when an absolute value of a difference between the first sensing time of the first sensing event in the far-field electrocardiogram and the second sensing time of the second sensing event in the *in vivo* near-field myocardial electrocardiogram is within a preset range;
when the first sensing event in the far-field electrocardiogram is generated by ventricular activation originating from atrial conduction, the preset range is greater than or equal to 0 ms and less than or equal to 120 ms; when the R wave in the far-field electrocardiogram is generated by ventricular activation originating from a ventricle or by a ventricular pacing pulse, the preset range is greater than or equal to 0 ms and less than or equal to 250 ms.

9. The medical instrument according to claim 8, wherein the step of acquiring the first sensing time of the R wave in the far-field electrocardiogram and the second sensing time of the R wave in the *in vivo* near-field myocardial electrocardiogram corresponding to the R wave in the far-field electrocardiogram specifically further comprises:
taking the first sensing time as a first time point, and taking a time point from corresponding first set duration before the first time point as a time reference zero point, acquiring the second sensing event in the *in vivo* near-field myocardial electrocardiogram after the time reference zero point, the first set duration being greater than or equal to 10 ms and less than or equal to 120 ms;
or,
taking the second sensing time as a second time point, and taking a time point from corresponding second set duration before the second time point as a time reference zero point, acquiring the first sensing event in the far-field electrocardiogram after the time reference zero point, the second set duration being greater than or equal to 10 ms and less than or equal to 120 ms.

10. The medical instrument according to claim 9, wherein the step of acquiring the first sensing time of the R wave in the far-field electrocardiogram and the second sensing time of the R wave in the *in vivo* near-field myocardial electrocardiogram corresponding to the R wave in the far-field electrocardiogram further comprises:
acquiring a sensing time window corresponding to the first sensing event in the far-field electrocardiogram;
judging whether the second sensing time or the first sensing time falls within the sensing time window;
if yes, determining that the first sensing event in the far-field electrocardiogram corresponds to the second sensing event in the *in vivo* near-field myocardial electrocardiogram, and that both the first sensing event and the second sensing event are R waves; if not, determining that the first sensing event in the far-field electrocardiogram does not correspond to the second sensing event in the *in vivo* near-field myocardial electrocardiogram, and that one of the first sensing event and the second sensing event is not an R wave, and the control device being configured not to deliver the CCM pulse stimulation to the myocardial position.

11. The medical instrument according to claim 10, wherein
when the first sensing time is taken as the first time point, and the first sensing event is an R wave, the step of judging whether the second sensing time or the first sensing time falls within the sensing time window comprises: judging whether the second sensing time falls within the sensing time window;
when the second sensing time is taken as the second time point, and the second sensing event is an R wave, the step of judging whether the second sensing time or the first sensing time falls within the sensing time window comprises: judging whether the first sensing time falls within the sensing time window.

12. The medical instrument according to claim 10, wherein
when the first sensing event in the far-field electrocardiogram is ventricular activation which does not originate from ventricular pacing, if the first sensing time is earlier than the second sensing time, a starting point of the sensing time window corresponding to the first sensing event in the far-field electrocardiogram is determined based on the first sensing time; if the second sensing time is earlier than the first sensing time, the starting point of the sensing time window corresponding to the first sensing event in the far-field electrocardiogram is determined based on the second sensing time;
when the first sensing event in the far-field electrocardiogram is generated by the ventricular pacing pulse, the starting point of the sensing time window corresponding to the first sensing event in the far-field electrocardiogram is determined based on a delivery time of the pacing pulse to the ventricle, and the delivery time of the pacing pulse is considered as the first sensing time.

13. The medical instrument according to claim 10, wherein the sensing time window has a start time earlier than or equal to the first sensing time or the second sensing time by a fourth preset duration, and the deliverable pulse time window has a second preset length; the sensing time window has a first preset length, and the second preset length is greater than the first preset length; the fourth preset duration is greater than or equal to 0 ms and less than or equal to 50 ms.

14. The medical instrument according to claim 10, wherein the sensing time window has a first preset length, and when the R wave in the far-field electrocardiogram is generated by ventricular activation originating from atrial conduction, the first preset length has a range of greater than or equal to 60 m and less than 120 ms; when the R wave in the far-field electrocardiogram is generated by ventricular activation originating from the ventricle or by the ventricular pacing pulse, the first preset length is greater than or equal to 160 m and less than or equal to 250 ms; or, the first preset length is determined through program control.

15. The medical instrument according to claim 10, wherein the number of the ventricular electrode leads is multiple and the ventricular electrode leads are arranged at multiple different ventricular myocardial positions, and the number of the *in vivo* near-field myocardial electrocardiograms and the second sensing events is multiple; the control device is further configured to execute the following steps:
presetting set sensing parameters corresponding to R waves at different myocardial positions, wherein the set sensing parameter comprises a set sensing time and/or a set sensing occurrence sequence;
setting all other remaining second sensing events as R waves when a first second sensing event falling within the sensing time window is an R wave; or,
setting all other remaining second sensing events as R waves when a last second sensing event falling within the sensing time window is an R wave.

16. The medical instrument according to claim 4, wherein the step of determining whether to deliver the CCM pulse stimulation to the myocardial position according to the pulse delivery start time and the deliverable pulse time window specifically comprises:
delivering the CCM pulse stimulation to the myocardial position if the pulse delivery start time falls within the deliverable pulse time window; and if not, not delivering the CCM pulse stimulation to the myocardial position.

17. The medical instrument according to claim 16, wherein the number of the ventricular electrode leads is multiple and the ventricular electrode leads are arranged at multiple different myocardial positions, and the number of the *in vivo* near-field myocardial electrocardiograms and the second sensing events is multiple; the control device is further configured to execute the following steps:
setting a pulse stimulation delivery sequence corresponding to the R waves at different myocardial positions, and delivering the CCM pulse stimulation to the different myocardial positions according to the pulse stimulation delivery sequence.

18. The medical instrument according to claim 16, wherein
when the R wave in the far-field electrocardiogram is ventricular activation which does not originate from ventricular pacing, a starting point of the deliverable pulse time window is determined based on the first sensing time of the R wave in the far-field electrocardiogram, or, based on the second sensing time, determined according to the first sensing time, of the corresponding R wave in the *in vivo* near-field myocardial electrocardiogram;
when the R wave in the far-field electrocardiogram is generated by a ventricular pacing pulse, the starting point of the deliverable pulse time window is determined based on a delivery time of the pacing pulse to the ventricle, and the delivery time of the pacing pulse is considered as the first sensing time.

19. The medical instrument according to claim 18, wherein the deliverable pulse time window has a second preset length, the second preset length has a range of greater than or equal to 150 ms and less than or equal to 300 ms, or the second preset length is determined through program control; the starting point of the deliverable pulse time window is earlier than the first sensing time or the second sensing time by a fourth preset duration, and the fourth preset duration is greater than or equal to 0 ms and less than or equal to 50 ms.

20. The medical instrument according to claim 18, wherein the control device is further configured to execute the following steps:
when the second sensing time is earlier than the first sensing time, determining the starting point of the deliverable pulse time window corresponding to the R wave in the *in vivo* near-field myocardial electrocardiogram according to the second sensing time, and determining the pulse delivery start time according to the first sensing time or the second sensing time;
when the second sensing time is later than the first sensing time, determining the starting point of the deliverable pulse time window corresponding to the R wave in the *in vivo* near-field myocardial electrocardiogram according to the first sensing time, and determining the pulse delivery start time according to the first sensing time or the second sensing time.

21. The medical instrument according to claim 4, wherein the step of determining whether to deliver the CCM pulse stimulation to the myocardial position according to the pulse delivery start time and the deliverable pulse time window specifically comprises:
determining a pulse delivery stop time according to the pulse delivery start time;
delivering the CCM pulse stimulation to the myocardial position if both the pulse delivery start time and the pulse delivery stop time fall within the deliverable pulse time window, and if not, not delivering the CCM pulse stimulation to the myocardial position.

22. The medical instrument according to claim 4, wherein the step of determining whether to deliver the CCM pulse stimulation to the myocardial position according to the pulse delivery start time and the deliverable pulse time window specifically comprises:
determining a pulse delivery stop time according to the pulse delivery start time and a preset pulse parameter;
redetermining the pulse parameter if the pulse delivery start time falls within the deliverable pulse time window, and the pulse delivery stop time does not fall within the deliverable pulse time window, so that both the pulse delivery start time and the pulse delivery stop time fall within the deliverable pulse time window, and delivering the CCM pulse stimulation to the myocardial position according to the redetermined pulse parameter;
not delivering the CCM pulse stimulation to the myocardial position if the pulse delivery start time does not fall within the deliverable pulse time window.

23. The medical instrument according to claim 4, wherein the step of determining whether to deliver the CCM pulse stimulation to the myocardial position according to the pulse delivery start time and the deliverable pulse time window specifically comprises:
determining a pulse delivery stop time according to the pulse delivery start time and a preset pulse parameter if the pulse delivery start time falls within the deliverable pulse time window;
judging whether the pulse delivery stop time falls within the deliverable pulse time window;
if yes, delivering the CCM pulse stimulation;
if not, not delivering the CCM pulse stimulation, or, redetermining the pulse parameter, so that the pulse delivery stop time falls within the deliverable pulse time window; and delivering the CCM pulse stimulation according to the redetermined pulse parameter.

24. The medical instrument according to claim 4, wherein the control device is configured to execute the following steps:
judging whether a ventricle has been captured if it is determined that a pacing pulse has been delivered to the myocardial position;
if yes, determining whether to deliver the CCM pulse stimulation to the myocardial position according to the pulse delivery start time and the deliverable pulse time window, wherein the deliverable pulse time window corresponding to the R wave in the *in vivo* near-field myocardial electrocardiogram is determined based on a delivery time of the pacing pulse to the ventricle;
if not, determining whether to deliver the CCM pulse stimulation to the myocardial position according to the pulse delivery start time and the deliverable pulse time window, wherein the deliverable pulse time window corresponding to the R wave in the *in vivo* near-field myocardial electrocardiogram is determined based on the first sensing time of the R wave in the far-field electrocardiogram, or, based on the second sensing time of the R wave in the *in vivo* near-field myocardial electrocardiogram.

25. The medical instrument according to claim 24, wherein the step of judging whether the ventricle has been captured specifically comprises: judging whether the ventricle has been captured based on the far-field electrocardiogram.

26. The medical instrument according to claim 1, wherein the step of delivering the CCM pulse stimulation to the myocardial position specifically comprises:
delivering the CCM pulse stimulation to the myocardial position if a current heart rate parameter is detected to be within a preset range.

27. The medical instrument according to claim 1,
wherein the CCM pulse stimulation is delivered during at least one of the following ventricular electrical activities:
a sinus beat, a ventricular beat originating from atrial conduction, a ventricular beat originating from a ventricle, and a ventricular beat originating from ventricular pacing.
